# EUROPEAN PATENT APPLICATION

(11) **EP 4 019 035 A1**
(43) Date of publication of application: **29.06.2022**
(21) Application number: 20217170.8
(22) Date of filing: 23.12.2020
(51) Int. Cl.: A61K 38/16, C07K 14/78, A61P 9/00, A61P 9/04, C12N 15/63, A61P 9/10

(54) **NOVEL RECOMBINANT FRAGMENTS OF FIBRILLIN-1 AND METHODS OF USE THEREOF**

(71) Applicant: INSTITUT NATIONAL DE LA SANTE ET DE LA RECHERCHE MEDICALE - INSERM, 75013 Paris (FR); UNIVERSITE DE BORDEAUX, 33000 Bordeaux (FR); The Royal Institution for the Advancement of Learning / McGill University, Montréal, Québec H3A 0G4 (CA)
(72) Inventor: GENOT, Elisabeth, 33400 Talence (FR); ALONSO, Florian, 33600 Pessac (FR); REINHARDT, Dieter, Montreal, Quebec H4B 1X9 (CA)
(74) Representative: Lecca, Patricia S.

(57) **Abstract**

The present invention relates to novel recombinant fragments derived from the fibrillin-1 protein, which are particularly useful for their pro-angiogenic and vessel normalization properties, pharmaceutical compositions comprising said fragments and methods of use for the prevention and/or treatment of vascular diseases.

## Description

### FIELD OF THE INVENTION

The present invention relates to novel recombinant fragments derived from the fibrillin-1 protein, which are particularly useful for their pro-angiogenic and vessel normalization properties, pharmaceutical compositions comprising said fragments and methods of use for the prevention and/or treatment of vascular diseases.

### BACKGROUND OF THE INVENTION

Fibrillin-1 is a large cysteine rich-glycoprotein of approximately 350 kDa, with a modular organization dominated by multiple tandem repeats of the calcium binding epidermal growth factor-like (cbEGF) domain interrupted by 8 cysteine containing domains. Fibrillin-1 as well as fibrillin-2, and fibulin-4 are molecular components of elastic fibers and microfibrils. Elastic fibers and microfibrils are stable structures especially abundant in the connective tissues of blood vessels, lung, skin, ligaments and tendons, and the eye. More specifically, fibrillin-1 is an extracellular matrix (ECM) protein that assembles into microfibrils which provide a scaffold for elastin deposition. Monomers are arranged head-to-tail, forming the characteristic beads-on-a-string microfibril structure. The protein also interacts with other ECM components, including MAGP1 and MAGP2, fibronectin, versican, perlecan, several fibulins and elastin, with several ADAMTS proteins and with integrins (α5β1, αvβ3, αvβ6, and α8β1). Fibrillin-1 functions also as a repository for latent transforming growth factor beta (TGFβ through its interaction with the TGFβ binding proteins (LTBPs), and for several bone morphogenic proteins (BMPs) through direct interactions. Consequently, mutations affecting the structure, assembly or stability of fibrillin-1 microfibrils can impact biochemical tissue properties, cell-matrix interactions or cytokine bioavailability. In addition, fibrillin-1 mutations are expected to affect the normal mechanosensing of cells. Fibrillin-1 contributes thus in relaying extracellular information to cellular signalling and function.

Marfan syndrome (MFS; Online Mendelian Inheritance in Man [OMIM] #154700) is a hereditary connective tissue disease caused by mutations in the fibrillin-1 gene (FBN1). This syndrome is associated with severe cardiovascular, skeletal, and ocular defects. In MFS, the defective microfibrils affect the integrity of elastic tissues such as the aortic wall, resulting in a progressive enlargement of the aortic diameter leading to aneurysms and dissections. In the eye, defective fibrillin-1-rich ciliary zonules cause lens dislocation in more than half of the patients. Interestingly, MFS patients also have a higher incidence (up to 26%) of retinal detachment than the general population, and whose etiology is unknown. Murine models have been instrumental in advancing our understanding of MFS. Heterozygous mice with a substitution of an obligatory cysteinyl residue in a cbEGF motif in one allele (Fbn1C1041G/+) reproduce several aspects of human MFS. These mice develop proximal aortic aneurysms, mitral valve thickening, pulmonary alveolar septation defects, mild thoracic kyphosis, and skeletal myopathy.

Over the several decades, an increasing number of diseases have been shown to result from a deterioration and/or a rarefaction of blood vessels associated with an upstream vascular occlusion or with a pathological remodelling or with vascular cell diseases. Tissues deprived of blood and oxygen undergo ischemic necrosis or infarction with possible irreversible organ damage. Once the flow of blood and oxygen is restored to the organ or tissue (reperfusion), the organ does not immediately return to the normal pre-ischemic state. Reperfusion of the blood flow is necessary to resuscitate the ischemic or hypoxic tissue or organ. Timely reperfusion facilitates salvage of cells and decreases morbidity and mortality. In an effort to provide treatment for ischemia, a number of angiogenesis techniques are now in clinical trial, including gene therapy and the use of growth factors such as vascular endothelial growth factor (VEGF) or basic fibroblast growth factor (bFGF) to induce or augment collateral blood vessel production. However, most of the advanced stage candidates show poor results compared to results obtained in preclinical models. These disparities may result from resistance to growth factors, from poor control of their release, in particular in terms of doses and kinetics. For these approaches, the possible occurrence of undesirable effects as a risk of stimulating angiogenesis in inappropriate places, of promoting haemorrhages and the possibility of stimulating the growth of still undiagnosed tumours, should be taken into account. Accordingly, there is an unmet need for new safe and effective therapies.

The present invention provides efficient novel therapeutic options to stimulate the formation of blood vessels (pro-angiogenesis) and accelerate the revascularization of ischemic areas, the vascularisation of regenerated tissues during wound repair, as well as to provide protective effects on the arteriolar structure and integrity to improve vessel quality and structure for treating and/or preventing retinopathies, age-related macular degeneration (AMD), hemangiomas, varicose veins, aneurysms and cerebrovascular diseases/malformations, inflammation- and diabetes-induced vascular diseases.

### SUMMARY OF THE INVENTION

The present invention provides novel isolated fragments derived from the fibrillin-1 protein comprising the amino acid sequence at least 70%, at least 80%, at least 85%, at least 90%, at least 95% or 100% identical or homologous to the amino acid sequence as set forth in SEQ ID NO: 1. Said polypeptides or peptide fragments are particularly useful as medicament, and more particularly as a pro-angiogenic drug and/or as a vessel normalization agent.

The present invention also provides pharmaceutical compositions comprising said fragments and methods of use of said fragments to stimulate the formation of new blood vessels (pro-angiogenesis) and to improve the structure and integrity of existing mature differentiated vessels or arterioles.

The present invention further provides methods of use of said fragments for treating and/or preventing ischemia or ischemic diseases or vascular diseases, such as ischemic heart diseases, coronary arterial diseases, myocardial infarction, angina pectoris, ischemic cerebrovascular diseases, peripheral ischemic diseases or peripheral arterial diseases such as lower limb artery ischemic diseases or distal limb ischemic diseases, diabetic retinopathy, ocular ischemia, stimulating wound healing, particularly in diabetic patients, as well as for treating and/or preventing age-related macular degeneration (AMD), hemangiomas, varicose veins, aneurysms and cerebrovascular diseases/malformations, inflammation- and diabetes-induced vascular diseases.

### BRIEF DESCRIPTION OF THE FIGURES

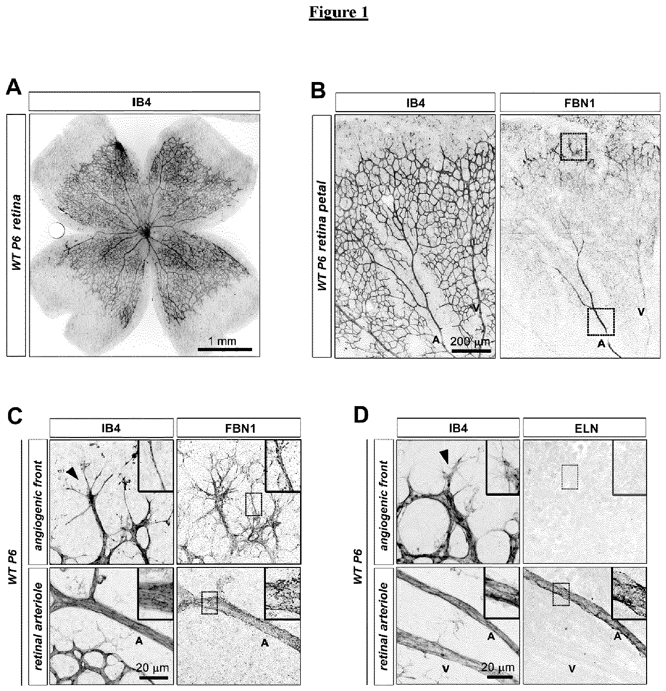

**Figure 1****:** Fibrillin-1 (FBN1) is expressed within the microvasculature of the developing mouse retina. **A.** Image of a flat-mounted P6 retina from WT mice stained for the endothelial marker Isolectin B4 (IB4) to visualize the developing microvascular network. **B.** FBN1 (right panel) and IB4 (left panel) staining in a single petal of a flat-mounted P6 retina from WT mice. **C.** High magnification images from the boxed regions in (**B**): angiogenic front (top) and arterioles (bottom): FBN1 (right panels) and IB4 (left panels). Higher magnification of corresponding inset (black boxes). (n = 10 WT mice). **D.** IB4 (left panels) and ELN (elastin) (right panels)-stained retinal vessels from P6 WT mice. Higher magnification of corresponding inset (black boxes). Black arrowheads; tip cells, A; arteriole and V; venule. (n = 3 WT mice).

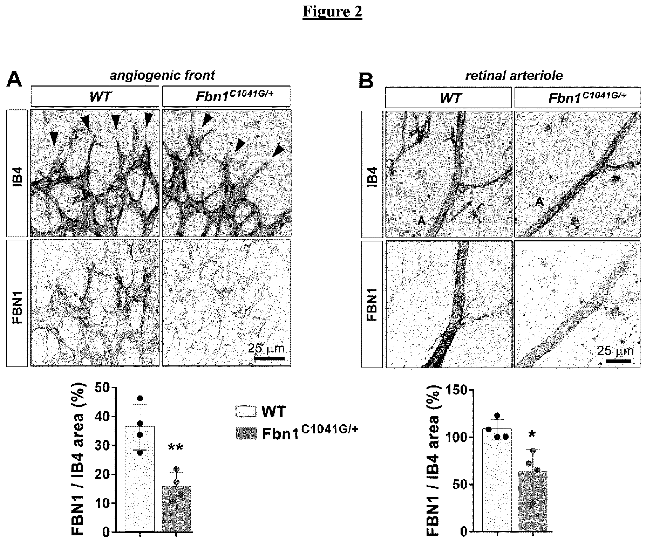

**Figure 2****:** A missense mutation in FBN1 results in a reduced FBN1 expression in the developing mouse retina. **A**. IB4 (top panels) and FBN1 (bottom panels) stained angiogenic front from Fbn1^{C1041G/+} mouse retinas at P6 (right panels) compared to WT (left panels). The quantification of the FBN1 positive area over the IB4 positive area is shown. (n =4 WT mice and 4 Fbn1^{C1041G/+}mice). **B**. Same experimental setup as in (**A**) analyzed at the level of arterioles. Black arrowheads; tip cells, A; arteriole. Data represent at least 3 independent experiments. Graphs are presented as mean values ± SD (bars) and individual values (dots). *P < 0.05; **P < 0.01 vs WT mice (Student's t test).

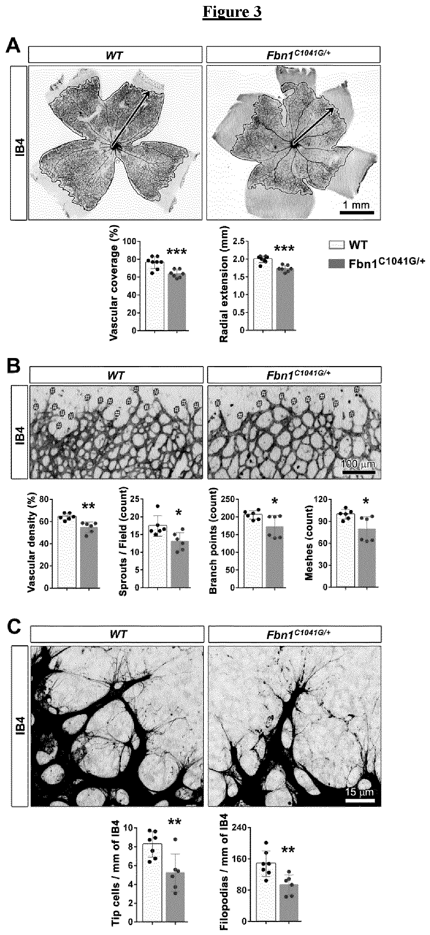

**Figure 3****:** A missense mutation in FBN1 impacts neoangiogenesis in the developing mouse retina. **A.** IB4 (black) stained retinal whole-mounts from Fbn1^{C1041G/+} mice at P6 (right panel) compared to WT (left panel). Quantifications of the vascular coverage (area delineated by the black line) and the radial extension are shown. (n = 8 WT and 7 Fbn1^{C1041G/+} mice). **B**. Higher magnification images at the angiogenic front from P6 WT and Fbn1^{C1041G/+} retinas. Quantitative assessments of the capillary density, the number of vascular sprouts (white #) and the number of branch points and meshes are shown. (n = 6 WT and 6 Fbn1^{C1041G/+} mice). **C.** High-resolution images of endothelial tip cells in P6 WT and Fbn1^{C1041G/+} retinas. Quantifications of the number of tip cells and filopodia per vessel front length are shown. (n = 7 WT and 6 Fbn-1^{C1041G /+}mice). Data represent at least 3 independent experiments. Graphs are presented as mean values ± SD (bars) and individual values (dots). *P < 0.05; **P < 0.01; ***P < 0.001 vs WT mice (Student's t tests).

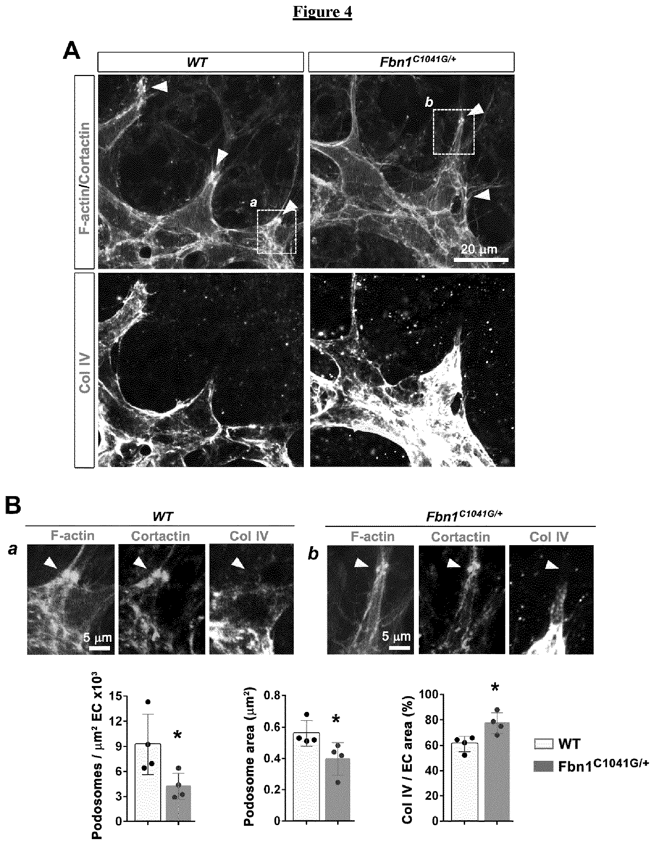

**Figure 4****:** FBN1 missense mutation impairs tip cell podosome formation and function. **A.** Representative images of the vascular front of WT and Fbn1^{C1041G/+/}Lifeact-EGFP P6 retinas labelled for cortactin (top panels), F-actin (Lifeact-EGFP, top panels) and Col IV (bottom panels). Tip cell podosomes (white arrowheads) are highlighted by F-actin and cortactin staining in areas devoid of Col IV staining. **B.** High magnification images shown as single staining from the 2 regions (a and b) boxed in (**A**). White arrowheads; podosomes. Quantitative analyses of podosome density, podosome area, and coverage of the neovesssels with Col IV are shown. (n = 4 WT/Lifeact-EGFP and 4 Fbn1^{C1041G/+} /Lifeact-EGFP mice). Data represent at least 3 independent experiments. Graphs are presented as mean values ± SD (bars) and individual values (dots). *P < 0.05 vs WT mice (Student's t tests).

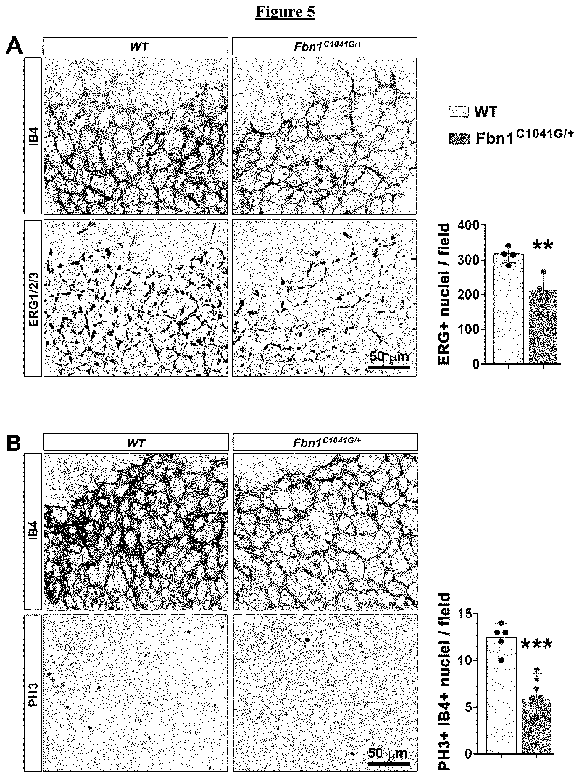

**Figure 5****:** Decreased EC proliferation in Fbn1^{C1141G/+} P6 retinas vs WT retinas. **A.** Dual labeling for IB4 (top panels) and the EC nuclei marker ERG1/2/3 (bottom panels) of the angiogenic front in P6 retinas from WT (left panels) and Fbn1^{C1041G/+} mice (right panels). Quantification of the number of IB4- and ERG1/2/3- double positive ECs per field is shown. (n = 4 WT and 4 Fbn1^{C1041G/+} mice). **B**. Same as in (**A**) for IB4 (top panels) and mitotic marker PH3 (bottom panels). Quantification of the number of PH3-positive ECs per field is shown. (n = 5 WT and 7 Fbn- 1^{C1041G/+}mice). Data are representative of 3 independent experiments. Graphs are presented as mean values ± SD (bars) and individual values (dots). **P < 0.01; ***P < 0.001 vs WT mice (Student's t tests).

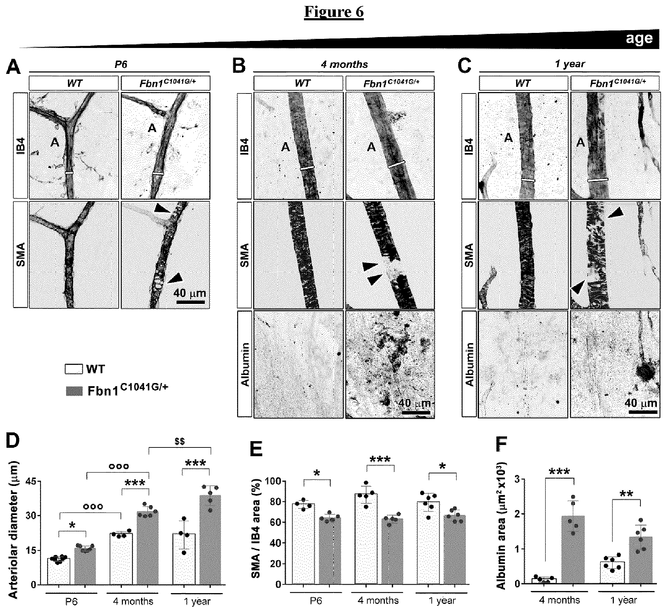

**Figure 6****:** FBN1 missense mutation progressively impairs retinal arteriole integrity and functionality. **A.** IB4 (top panels) and SMA (bottom panels) stained arteriole from P6 Fbn1^{C1041G/+} retinas (right panels) compared to WT (left panels). **B-C**. IB4 (top panels), SMA (intermediate panels) and Serum Albumin (bottom panels) stained arteriole in the retinas from 4- month (**B**) and 1-year old (**C**) WT (left panels) and Fbn1^{C1041G/+} (right panels) mice. **D-F.** Quantitative assessment of the retinal arteriolar diameter (white line in **A-C**) (**D**), SMA positive area over the IB4 positive area (**E**) and albumin positive area per field (**F**) according to age in WT and Fbn1^{C1041G/+} mice. A; arteriole. Data are representative of 3 independent experiments. Graphs are presented as mean values ± SD (bars) and individual values (dots). *P < 0.05; **P < 0.01; ***P < 0.001 vs respective WT mice. °°°P < 0.001 vs respective P6 mice. &&P < 0.01 vs respective 4-month-old mice (one-way ANOVA followed by Tukey's post-tests).

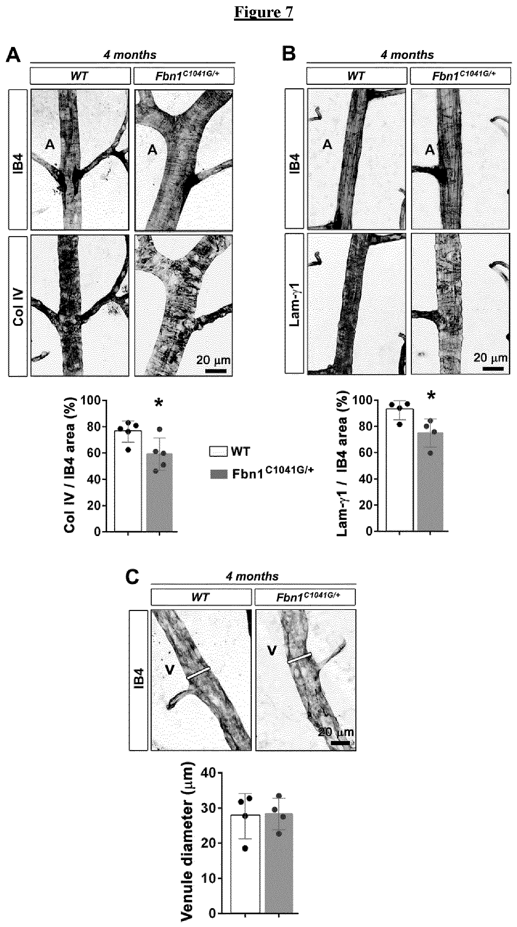

**Figure 7****:** Arteriolar basement membrane destabilization in Fbn1^{C1041G/+} retinas vs WT retinas. **A.** IB4 (top panels) and the basement membrane marker Col IV (bottom panels) stained arteriole from Fbn1^{C1041G/+} retinas at 4 months (right panels) compared to WT (left panels). Quantification of the Col IV positive area over the IB4 positive area is shown. (n = 5 WT and 5 Fbn1^{C1041G/+} mice). **B**. Same as in (**A**) for IB4 (top panels) and the basement membrane marker Laminin-gamma-1 (top panels). (n = 4 WT and 4 Fbn-1^{C1041G/+}mice). **C.** IB4 stained venule from Fbn1^{C1041G/+} retinas at 4 months (right panel) compared to WT (left panel). Quantification of the retinal venule diameter (white line) is shown. (n = 4 WT and 4 Fbn1^{C1041G/+}mice). A; arteriole and V; venule. Data are representative from 3 independent experiments. Graphs are presented as mean values ± SD (bars) and individual values (dots). *P < 0.05 vs WT mice (Student's t tests).

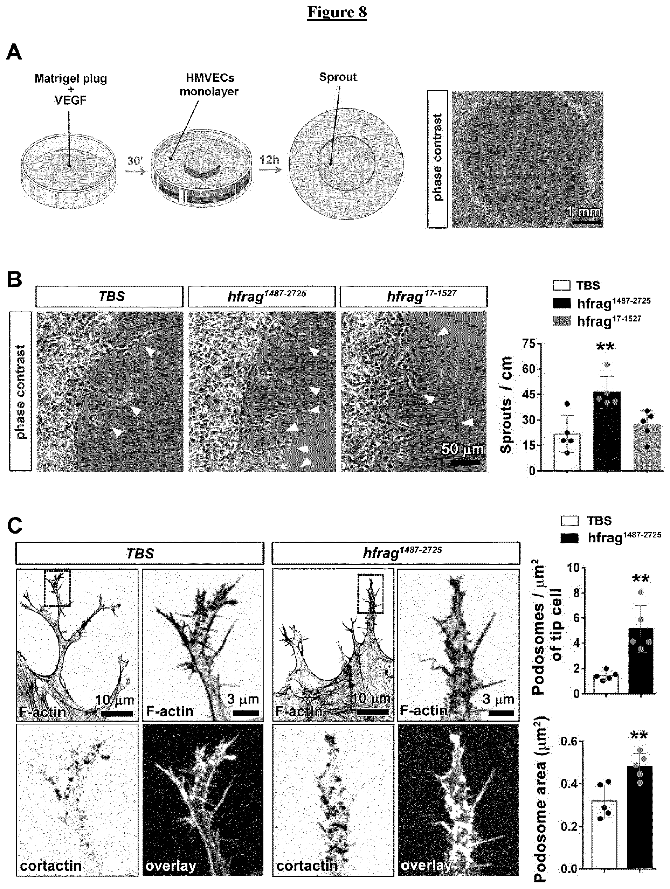

**Figure 8****:** *In vitro* effects of the human recombinant FBN1 fragments according to the invention on EC sprouting. **A.** Schematic representation of the *in vitro* angiogenesis invasion assay (AIA) and phase contrast picture showing the tip-cell-like cell phenotype of HMVECs exposed to the Matrigel and VEGF-A gradient in this setting. **B.** High magnification phase contrast images of HMVECs protruding into the Matrigel supplemented with either TBS (vehicule) (left panel), the human C-terminal FBN1 fragment (hfrag¹⁴⁸⁷⁻²⁷²⁵, middle panel) or the N-terminal FBN1 fragment (hfrag¹⁷⁻¹⁵²⁷, right panel). Quantitative analysis of EC sprouting (white arrowheads) under the different situations is shown. Data are representative from 5 independent experiments. Graphs are presented as mean values ± SD (bars) and individual values (dots). **P < 0.01 vs TBS. (one-way ANOVA followed by Tukey's post-test). **C.** F-actin/cortactin double staining of tip-cell-like ECs protruding into the Matrigel supplemented with either TBS (left panels) or the hfrag¹⁴⁸⁷⁻²⁷²⁵ fragment (right panels), showing podosomes (black foci) at the cell protrusions. Higher magnification of corresponding inset (black boxes, inverted signal). Quantitative analysis of the number and size of podosomes are shown. Data are representative from 5 independent experiments. Graphs are presented as mean values ± SD (bars) and individual values (dots). **P < 0.01 vs TBS. (Student's t tests).

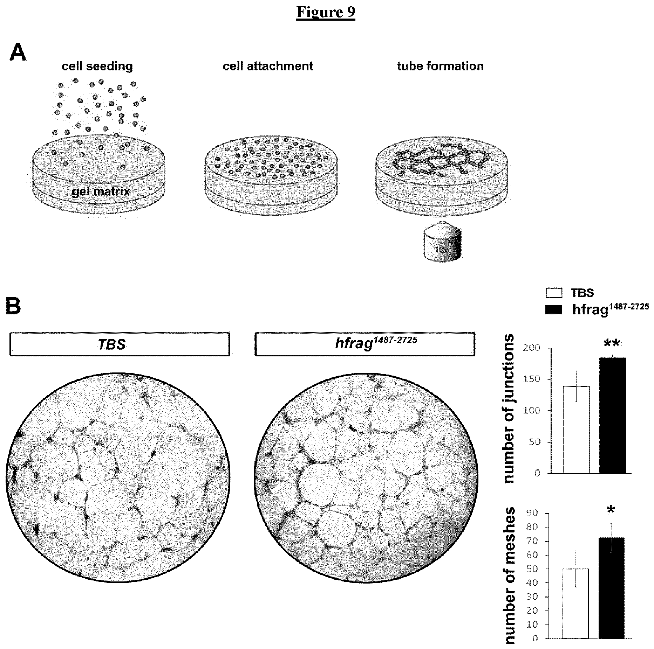

**Figure 9****:** *In vitro* effects of the human recombinant FBN1 fragments according to the invention on endothelial cell (EC) branching. **A.** Schematic representation of the *in vitro* tube formation assay. **B.** Low magnification phase contrast images of HMVECs capillary-like structures formed into the Matrigel supplemented with either TBS (left panel) or the human FBN1 fragment, *e*.*g*., hfrag¹⁴⁸⁷⁻²⁷²⁵, right panel. Quantitative analysis of the number of junctions (branch points) and the number of meshes are shown. Data are representative from 5 independent experiments. Graphs are presented as mean values ± SD (bars). *P < 0.05; **P < 0.01 vs TBS. (Student's t tests).

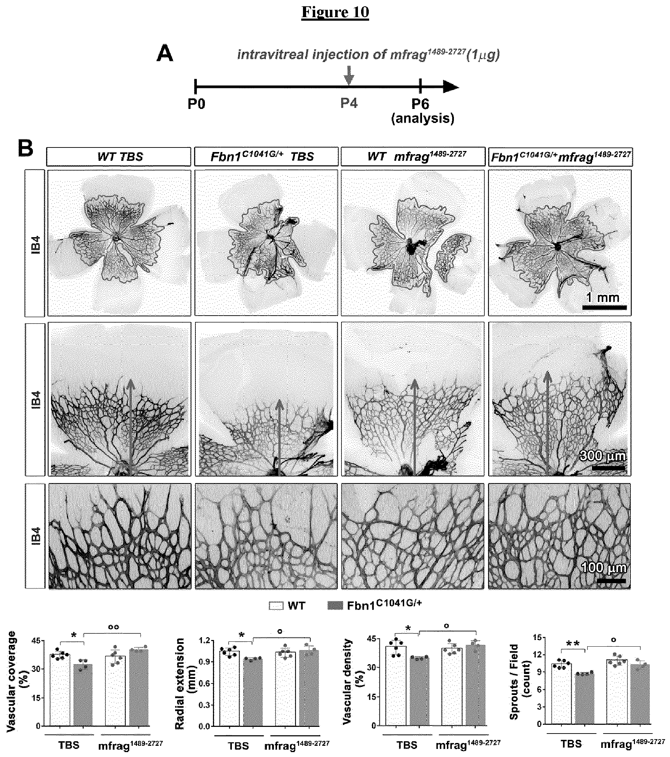

**Figure 10****:** *In vivo* effects of the murine recombinant fibrillin-1 fragment according to the invention, *e*.*g*., mfrag¹⁴⁸⁹⁻²⁷²⁷. **A.** Schematic representation of the protocol of intravitreal administration of the mfrag¹⁴⁸⁹⁻²⁷²⁷ fragment. **B.** IB4 (black) stained retinal whole-mounts from P6 WT and Fbn1^{C1041G/+} mice intravitreally injected with TBS (vehicle) or the mfrag¹⁴⁸⁹⁻²⁷²⁷ fragment at P4. Quantifications and representative images of the vascular coverage (delimited by the black line, top panels), radial extension (black arrow, middle panels), capillary density and sprouting (bottom panels) are shown. (n = 6 TBS-injected WT; 6 mfrag¹⁴⁸⁹⁻²⁷²⁷-injected WT ; 4 TBS- injected Fbn1^{C1041G/+} ; 4 mfrag¹⁴⁸⁹⁻²⁷²⁷-injected Fbn1^{C1041G/+} mice). Data are representative from 3 independent experiments. Graphs are presented as mean values ± SD (bars) and individual values (dots). *P < 0.05; **P < 0.01 vs respective WT mice. °P < 0.05; °°P < 0.01 vs respective TBS-injected mice (one-way ANOVA followed by Tukey's post-test).

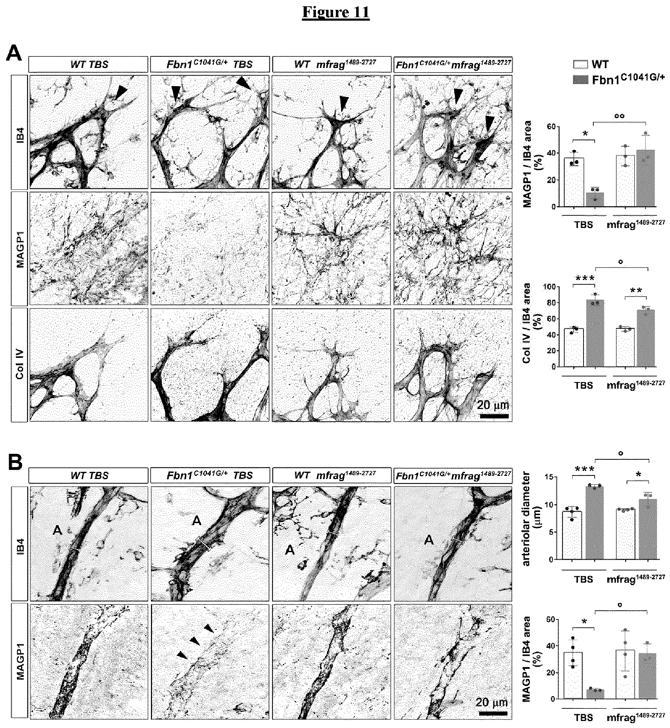

**Figure 11****:** *In vivo* effects of the mfrag¹⁴⁸⁹⁻²⁷²⁷ fragment (continued) **A.** IB4 (top panels), MAGP1 (intermediate panels) and Col IV (bottom panels) stained angiogenic front from P6 WT and Fbn1^{C1041G/+} mice intravitreally injected with TBS or the mfrag¹⁴⁸⁹⁻²⁷²⁷ fragment at P4. Black arrowheads; tip cells. Quantifications of the MAGP1 and Col IV positive areas over the IB4 positive area are shown. (n=3 TBS- and 3 mfrag¹⁴⁸⁹⁻²⁷²⁷-injected WT and 3 TBS- and 3 mfrag¹⁴⁸⁹⁻²⁷²⁷-injected Fbn1^{C1041G/+} mice). **B.** IB4 (top panels) and MAGP1 (bottom panels) stained retinal arterioles from P6 WT and Fbn1^{C1041G/+} mice intravitreally injected with TBS or the mfrag¹⁴⁸⁹⁻²⁷²⁷ fragment at P4. A; arteriole. Black arrowheads; loss of MAGP1. Quantifications of the arteriolar diameter and the MAGP1 positive areas over the IB4 positive area are shown. (n=4 TBS- and 4 mfrag¹⁴⁸⁹⁻²⁷²⁷-injected WT and 3 TBS- and 3 mfrag¹⁴⁸⁹⁻²⁷²⁷-injected Fbn1^{C1041G/+} mice). Data are representative from 3 independent experiments. Graphs are presented as mean values ± SD (bars) and individual values (dots). *P < 0.05; **P < 0.01; ***P < 0.001 vs respective WT mice. °P < 0.05; °°P < 0.01 vs respective TBS-injected mice (one-way ANOVA followed by Tukey's post-test).

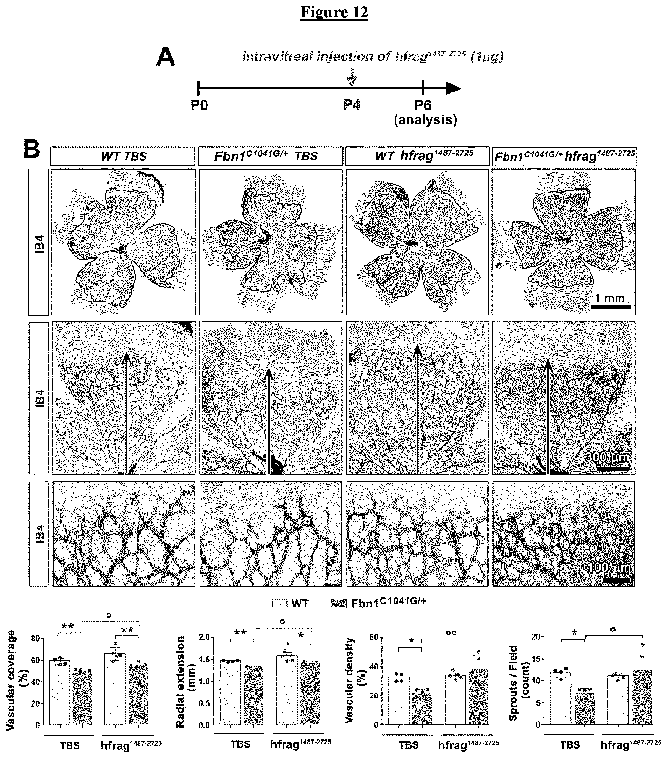

**Figure 12****:** Effects of the human recombinant FBN1 C-terminal fragment (hfrag¹⁴⁸⁷⁻²⁷²¹) on the developing mouse retinal vasculature, *in vivo.* **A.** Schematic representation of the protocol of intravitreal administration of the hfrag¹⁴⁸⁷⁻²⁷²⁵ fragment. **B.** IB4 (black) stained retinal whole-mounts from P6 WT and Fbn1^{C1041G/+} mice intravitreally injected with TBS or the hfrag¹⁴⁸⁷⁻²⁷²⁵ fragment at P4. Quantifications and representative images of the vascular coverage (delimited by the black line, top panels), radial extension (black arrow, middle panels), capillary density and sprouting (bottom panels) are shown. (n = 4 TBS- injected WT; 5 hfrag¹⁴⁸⁷⁻²⁷²⁵-injected WT; 5 TBS- injected Fbn1^{C1041G/+} ; 5 hfrag¹⁴⁸⁷⁻²⁷²⁵-injected Fbn1^{C1041G/+} mice). Data are representative from 3 independent experiments. Graphs are presented as mean values ± SD (bars) and individual values (dots). *P < 0.05; **P < 0.01 vs respective WT mice. °P < 0.05; °°P < 0.01 vs respective TBS-injected mice (one-way ANOVA followed by Tukey's post-test).

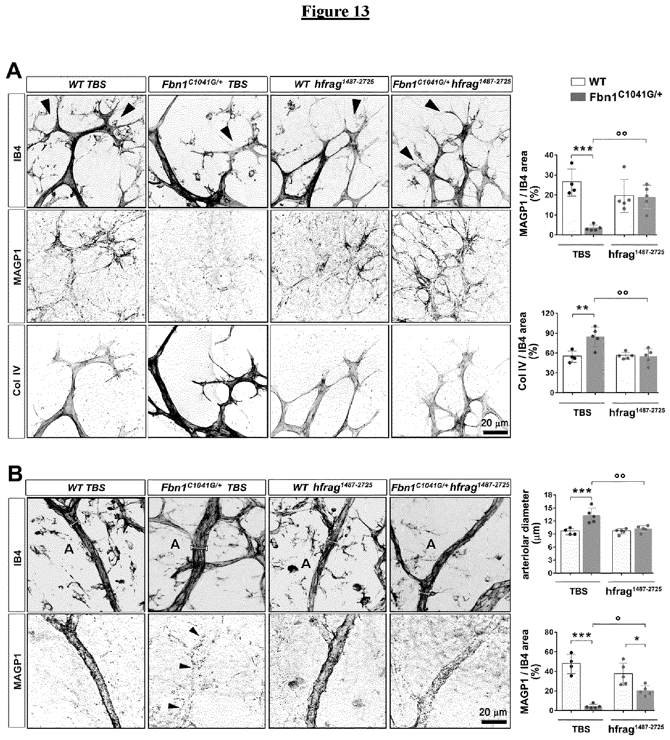

**Figure 13****:** Effects of the human recombinant FBN1 C-terminal fragment (hfrag¹⁴⁸⁷⁻²⁷²⁵) on the developing mouse retinal vasculature, *in vivo* (continued). **A.** IB4 (top panels), MAGP1 (intermediate panels) and Col IV (bottom panels) stained angiogenic front from P6 WT and Fbn1^{C1041G/+} mice intravitreally injected with TBS or the hfrag¹⁴⁸⁷⁻²⁷²⁵ fragment at P4. Black arrowheads; tip cells. Quantifications of the MAGP1 and Col IV positive areas over the IB4 positive area are shown. (n=4 TBS- and 5 hfrag¹⁴⁸⁷⁻²⁷²⁵-injected WT and 5 TBS- and 5 hfrag¹⁴⁸⁷⁻²⁷²⁵-injected Fbn1^{C1041G/+} mice). **B.** IB4 (top panels) and MAGP1 (bottom panels) stained retinal arterioles from P6 WT and Fbn1^{C1041G/+} mice intravitreally injected with TBS or the hfrag¹⁴⁸⁷⁻²⁷²⁵ fragment at P4. A; arteriole. Black arrowheads; loss of MAGP1. Quantifications of the arteriolar diameter and the MAGP1 positive areas over the IB4 positive area are shown. (n=4 TBS- and 5 hfrag¹⁴⁸⁷⁻²⁷²⁵ -injected WT and 5 TBS- and 5 hfrag¹⁴⁸⁷⁻²⁷²⁵ injected Fbn1^{C1041G/+} mice). Data are representative from 3 independent experiments. Graphs are presented as mean values ± SD (bars) and individual values (dots). *P < 0.05; **P < 0.01; ***P < 0.001 vs respective WT mice. °P < 0.05; °°P < 0.01 vs respective TBS-injected mice (one-way ANOVA followed by Tukey's post-test).

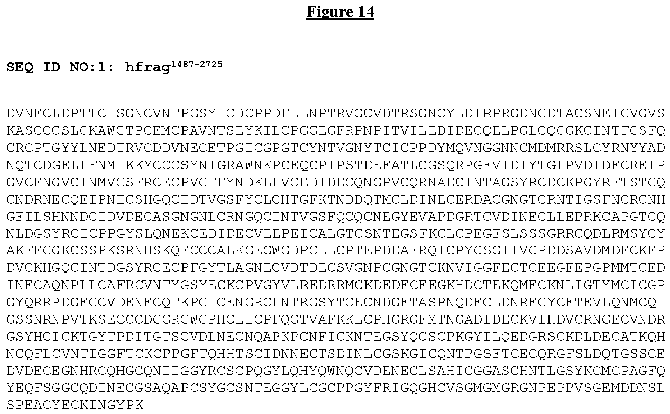

**Figure 14****:** provides the amino acid sequence of fibrillin-1 fragment as set forth in SEQ ID NO: 1.

### DETAILED DESCRIPTION

The present invention relates generally to novel and recombinant fragments derived from the C-terminal part of the fibrillin-1 protein, and further to the unexpected pro-angiogenic and vessel normalization properties of the said recombinant fragments. The present invention thus provides with isolated recombinant polypeptide fragments comprising 1239 amino acids and derived from amino acid residue 1487 to amino acid residue 2725 (and thus designated herein below as hfrag¹⁴⁸⁷⁻²⁷²⁵), by reference to the amino acid sequence of the human fibrillin-1 protein or derived from amino acid residue 1489 to amino acid residue 2727 (and thus designated herein below as mfrag¹⁴⁸⁹⁻²⁷²⁷) by reference to the murine fibrillin-1 protein.

More precisely, fibrillin-1 fragments according to the present invention comprising amino acid sequence as set forth in SEQ ID: 1, or at least 70%, at least 80% at least 90%, or at least 95% identical or homologous to the amino acid sequence as set forth in SEQ ID No: 1, precursors, salts, and/or derivatives thereof.

Fibrillin-1 fragments according to the present may be derived from the human or murine fibrillin-1 protein and comprise the amino acid sequence as set forth in SEQ ID NO: 1. They may be derived from fibrillin-1 proteins of other species, that have identity of sequences with SEQ ID NO: 1 and similar therapeutical activities as described below.

The bed rock principle of the present invention is based on the unexpected findings of the angiogenic properties of the active recombinant polypeptide fragments derived from fibrillin-1 to stimulate the formation of blood vessels, promote the microvascular integration of biological implants within the host tissue, and increase the structure and integrity of mature differentiated vessels or arterioles. Said fragments are particularly useful for affecting angiogenesis and conditions associated with angiogenesis and/or or vasculogenesis.

Applicants discovered that fibrillin-1 fragments as described above (corresponding to the C-terminal region containing the RGD cell binding domain) showed pro-angiogenic activity in *in vitro* angiogenesis assays when added to the basement material-like matrix element (Matrigel). More specifically, polypeptide fragments according to the present invention were useful for increasing the density of the vascular network in a model of microvascular tube formation. This activity was compatible with the role of fibrillin-1 in regulating the complexity of the network identified *in vivo* in the neonatal retina of mice. The hfrag¹⁴⁸⁷⁻²⁷²⁵ fragment was showed to stimulate the extension of vascular sprouts in a 3D invasion assay where microvascular endothelial cells are facing a matrix environment, compatible with the role of fibrillin-1 in endothelial sprouting and the maintenance of the tip cell phenotype identified *in vivo* in the neonatal retina of mice. Applicants also found that either the hfrag¹⁴⁸⁷⁻²⁷²⁵ fragment or a murine fragment, *e*.*g*., mfrag¹⁴⁸⁹⁻²⁷²⁷ corresponding to the hfrag¹⁴⁸⁷⁻²⁷²⁵ fragment promoted the growth of the vascular network in the retina after intraocular injection in mice carrying a fibrillin-1 "missense" mutation C1041G. Furthermore, Applicants discovered that the fibrillin-1 fragments according to the present invention corrected the increased arteriolar diameter and the reduced MAGP1 expression observed in C1041G fibrillin-1 mutant mice. It has thus been established for the first time that administration of these fibrillin-1 fragments according to the invention, not only allowed stimulating angiogenesis *in vivo,* but also had protective effects on the integrity of mature vessels such as arterioles, in particular by improving vessel's structure and quality.

Recombinant protein, cell culture, and molecular cloning techniques utilized in the present invention are standard procedures, well known to those skilled in the art. Such techniques are described and explained throughout the literature in sources such as, J. Perbal, A Practical Guide to Molecular Cloning, John Wiley and Sons (1984), J. Sambrook et al., Molecular Cloning: A Laboratory Manual, Cold Spring Harbour Laboratory Press (1989), T. A. Brown (editor), Essential Molecular Biology: A Practical Approach, Volumes 1 and 2, IRL Press (1991), D. M. Glover and B. D. Hames (editors), DNA Cloning: A Practical Approach, Volumes 1-4, IRL Press (1995 and 1996), and F. M. Ausubel et al. (editors), Current Protocols in Molecular Biology, Greene Pub. Associates and Wiley-Interscience (1988, including all updates until present), Ed Harlow and David Lane (editors) Antibodies: A Laboratory Manual, Cold Spring Harbour Laboratory, (1988), and J. E. Coligan et al. (editors) Current Protocols in Immunology, John Wiley & Sons (including all updates until present).

The terms "protein," "polypeptide," and "peptide," used interchangeably herein, include polymeric forms of amino acids of any length, including coded and non-coded amino acids and chemically or biochemically modified or derivatized amino acids. The terms also include polymers that have been modified, such as polypeptides having modified peptide backbones. Proteins are said to have an "N-terminus" and a "C-terminus." The term "N-terminus" relates to the start of a protein or polypeptide, terminated by an amino acid with a free amine group (-NH2). The term "C-terminus" relates to the end of an amino acid chain (protein or polypeptide), terminated by a free carboxyl group (-COOH).

The term "isolated" with respect to proteins and nucleic acid includes proteins and nucleic acids that are relatively purified with respect to other bacterial, viral or cellular components that may normally be present in situ, up to and including a substantially pure preparation of the protein and the polynucleotide. The term "isolated" also includes proteins and nucleic acids that have no naturally occurring counterpart, have been chemically synthesized and are thus substantially uncontaminated by other proteins or nucleic acids, or has been separated or purified from most other cellular components with which they are naturally accompanied (e.g., other cellular proteins, polynucleotides, or cellular components). By an "isolated polynucleotide", including DNA, RNA, or a combination of these, single or double stranded, in the sense or antisense orientation or a combination of both, we mean a polynucleotide which is at least partially separated from the polynucleotide sequences with which it is associated or linked in its native state. Preferably, the isolated polynucleotide is at least 60% free, preferably at least 75% free, and most preferably at least 90% free from other components with which they are naturally associated.

"Sequence identity" or "identity" in the context of two polypeptide sequences refers to the residues in the two sequences that are the same when aligned for maximum correspondence over a specified comparison window. When percentage of sequence identity is used in reference to proteins, it is recognized that residue positions which are not identical often differ by conservative amino acid substitutions, where amino acid residues are substituted for other amino acid residues with similar chemical properties (e.g., charge or hydrophobicity) and therefore do not change the functional properties of the molecule. When sequences differ in conservative substitutions, the percent sequence identity may be adjusted upwards to correct for the conservative nature of the substitution. Sequences that differ by such conservative substitutions are said to have "sequence similarity" or "similarity." Means for making this adjustment are well known to those of skill in the art. Typically, this involves scoring a conservative substitution as a partial rather than a full mismatch, thereby increasing the percentage sequence identity. Thus, for example, where an identical amino acid is given a score of 1 and a non-conservative substitution is given a score of zero, a conservative substitution is given a score between zero and 1. The scoring of conservative substitutions is calculated, e.g., as implemented in the program PC/GENE (Intelligenetics, Mountain View, Calif.).

"Percentage of sequence identity" includes the value determined by comparing two optimally aligned sequences over a comparison window, wherein the portion of the amino acid sequence in the comparison window may comprise additions or deletions (i.e., gaps) as compared to the reference sequence (which does not comprise additions or deletions) for optimal alignment of the two sequences. The percentage is calculated by determining the number of positions at which the identical amino acid residue occurs in both sequences to yield the number of matched positions, dividing the number of matched positions by the total number of positions in the window of comparison, and multiplying the result by 100 to yield the percentage of sequence identity.

Unless otherwise stated, sequence identity/similarity values include the value obtained using GAP Version 10 using the following parameters: % identity and % similarity for a amino acid sequence, and thus sequence identity typically refer to sequences which have the stated value when assessed using ClustalW (Thompson et al., 1994, supra) with the following parameters: Pairwise alignment parameters -Method: accurate, Matrix: PAM, Gap open penalty: 10.00, Gap extension penalty: 0.10; Multiple alignment parameters -Matrix: PAM, Gap open penalty: 10.00, % identity for delay: 30, Penalize end gaps: on, Gap separation distance: 0, Negative matrix: no, Gap extension penalty: 0.20, Residue-specific gap penalties: on, Hydrophilic gap penalties: on, Hydrophilic residues: G, P, S, N, D, Q, E, K. Sequence identity at a particular residue is intended to include identical residues which have simply been derivatized.

Homologous sequences are mentioned herein. The homologous sequences may represent the therapeutic 'portion' sequence within the peptide or be the second (remainder) sequence in the peptide of the invention. The homologous sequences may be homologous of any of the specific sequences mentioned herein. A homologue has one or more (such as a least 2, 3, 4 or 5) additions and/or deletions and/or substitutions in comparison to the original sequence. The homologous sequence may comprise at least 70%, at least 75%, at least 80%, at least 85%, at least 87%, at least 90%, at least 95%, 96%, 97%, 98%, or at least 99% amino acid identity over the length of the original sequence or over 4, 6, 10, 15 or 20 amino acids of the original sequence.

The homologous sequence may have 1, 2, 3, 4, 5 or more, or up to 10 amino acid substitutions in comparison to the original sequence. The substitutions are preferably conservative substitutions. These may be made according to the Table 1 below. Amino acids in the same block in the second column and preferably in the same line in the third column may be substituted for each other.

**Table 1:**

| | | |
|---|---|---|
| ALIPHATIC | Non-polar | G A P |
| | | I L V |
| | Polar-uncharged | C S T M |
| | | N Q |
| | Polar-charged | D E |
| | | K R |
| AROMATIC | | H F W Y |

Preferably, the amino acid sequences of polypeptide fragments frag¹⁴⁸⁹⁻²⁷²⁷ have 75%, 80%, 85%, 87%, 90%, 93%, 95%, 96%, 97%, 98%, or 99% amino acid identity to SEQ ID NO: 1 and said fragments retain pro-angiogenic activity as showed by typical pro-angiogenic *in vitro* tests (such as for example Matrigel tests) and/or retain blood vessel normalization or stabilization activity as showed by analysis of the adult retinal vasculature (such as immunochemistry of mature retinal vasculature such as immunostaining of collagen IV and laminin and quantitative analysis thereof). Suitable levels of homology are discussed in the section below relating to homology and include for example analogues or conservative variants thereof. With regard to polypeptide fragments, it will be appreciated that % identity figures higher than those provided above will encompass preferred embodiments. Thus, where applicable, in light of the minimum % identity figures, it is preferred that the polypeptide fragments comprise an amino acid sequence which is at least 50%, more preferably at least 60%, more preferably at least 70%, more preferably at least 80%, more preferably at least 85%, more preferably at least 90%, more preferably at least 91%, more preferably at least 92%, more preferably at least 93%, more preferably at least 94%, more preferably at least 95%, more preferably at least 96%, more preferably at least 97%, more preferably at least 98%, more preferably at least 99%, more preferably at least 99.1%, more preferably at least 99.2%, more preferably at least 99.3%, more preferably at least 99.4%, more preferably at least 99.5%, more preferably at least 99.6%, more preferably at least 99.7%, more preferably at least 99.8%, and even more preferably at least 99.9% identical to the amino acid sequence as set forth in SEQ ID NO: 1.

Also included in the present invention are variants, or derivatives of polypeptides, and any combination thereof. The term "fragment" or "variant" when referring to polypeptides and proteins of the present invention include any polypeptides or proteins which retain at least some of the properties (e.g., pro-angiogenic activity and/or blood vessel normalization or stabilization activity) of the reference polypeptide fragment of SEQ ID NO: 1. Variants include fragments as described above with altered amino acid sequences due to amino acid substitutions, deletions, or insertions. Variants can be naturally or non-naturally occurring. Non-naturally occurring variants can be produced using art-known mutagenesis techniques. Variants can comprise conservative or non-conservative amino acid substitutions, deletions, or additions. "Derivatives" are polypeptide fragments which have been altered to exhibit additional features not found on the native polypeptide or protein.

The present invention thus provides isolated recombinant fibrillin-1 polypeptide fragments comprising amino acid sequence as set forth in SEQ ID: 1, or having at least 70%, at least 80% or at least 90%, or at least 95% amino acid identity to SEQ ID No: 1, and the uses of frag¹⁴⁸⁹⁻²⁷²⁷ as medicament. These novel recombinant fibrillin-1 fragments constitute efficient novel angiogenic agents having efficient pro-angiogenic as well as blood vessel normalization or stabilization activity, and are capable of stimulating revascularisation of ischemic tissues, such as for example vascularisation during wound repair, or vascularisation of biological implants in tissue bioengineering, as well as protecting or improving the arteriolar integrity and structure. These novel fibrillin-1 recombinant fragments thus represent new therapeutic angiogenesis strategies and drugs and are particularly useful as a pro-angiogenic drug and a vascular normalization or stabilization agent.

The present invention thus also relates to pharmaceutical compositions comprising said recombinant fibrillin-1 fragments as described above as active ingredients and methods of use thereof to stimulate the revascularization of ischemic tissues, vascularisation during wound healing as well as to improve vessel structure and integrity in retinopathies, age-related macular degeneration (AMD), hemangiomas, varicose veins, aneurysms and cerebrovascular diseases/malformations, inflammation- and diabetes-induced vascular diseases.

The present invention also has applications in the field of regenerative medicine, in particular in tissue bioengineering to build a vascular network within an implant, *i.e*., vascularize biological implants in tissue bioengineering, and/or to connect two vascular networks, that of the implant and that of the host. Indeed, the regeneration of large volumes within an adult tissue does not occur spontaneously. Regenerative medicine then uses tissue engineering to design biological substitutes for tissue repair. This method works satisfactorily for tissue engineering products intended for the regeneration of the skin or cartilage, which are naturally poorly vascularized. For other tissues that heavily depend on vascularization for their survival, the limiting step remains the integration of the implant relies on the connection of host/implant microvascular networks.

The pharmaceutical compositions may comprise a therapeutically effective amount of a polypeptide according to SEQ ID NO: 1 and at least one pharmaceutically acceptable carrier and/or vehicle. Said compositions may further comprise an effective amount of at least one therapeutic agent chosen among an anticoagulant, an anti-thrombotic agent, an anti-platelet agent, a fibrinolytic agent, a hypolipidemic agent, an antihypertensive agent, and/or an anti-ischemic agent.

The present invention thus provides methods of treating and/or preventing ischemic diseases, vascular diseases or stimulating wound healing, comprising administering to a subject in need therefor a therapeutically effective amount of fibrillin-1 polypeptide fragments as described above, a physiologically acceptable variant, fragment, precursor, salt, or derivative thereof.

By way of example of ischemic diseases, we may cite cardiovascular diseases or ischemic heart diseases, such as coronary arterial disease, coronary arterial thrombosis, myocardial infarction, angina pectoris, acute coronary syndrome, atrial fibrillation, ischemic sudden death, or transient ischemic attack. We may also cite peripheral ischemic diseases or peripheral arterial diseases such as peripheral occlusive arterial disease, lower limb artery ischemic diseases, distal limb ischemic diseases, thromboembolic disorders, venous thrombosis, deep vein thrombosis, thrombophlebitis, arterial embolism, or wound healing in diabetic patients.

Other ischemic diseases which may be advantageously treated and/or prevented by the administration of the polypeptide fragments or pharmaceutical compositions according to the present invention include diabetic retinopathy, ocular ischemia, ischemic cerebrovascular diseases, cerebral arterial thrombosis, cerebral embolism, kidney embolism, pulmonary embolism, and ischemia and/or thrombosis resulting from (a) prosthetic valves or other implants, (b) indwelling catheters, (c) stents, (d) cardiopulmonary bypass, (e) hemodialysis, or (f) other procedures in which blood is exposed to an artificial surface that promotes thrombosis.

As indicated above, one objective of the invention is therefore to use the pro-angiogenic properties of a recombinant fibrillin-1 fragments as described above to stimulate the formation of blood vessels (angiogenesis) and thereby accelerate the revascularization of ischemic areas, the vascularisation of regenerated tissues during wound repair, as well as to provide protective effects on the arteriolar structure and integrity to improve vessel quality and structure.

The present invention thus also provides with use of fibrillin-1 fragments as described above and methods of treating and/or preventing diseases associated with loss of vessel integrity and situations wherein vessel normalization should be promoted, in particular for treating and/or preventing age-related macular degeneration (AMD), hemangiomas, varicose veins, aneurysms and cerebrovascular diseases/malformations, inflammation- and diabetes-induced vascular diseases.

Also within the scope are combination therapies, wherein methods of treating and/or preventing ischemia or ischemic diseases or vascular diseases comprise further administering a further pro-angiogenic agent, an anticoagulant, an anti-thrombotic agent, an anti-platelet agent, a fibrinolytic agent, a hypolipidemic agent, an antihypertensive agent, and/or an anti-ischemic agent.

Possible routes of administration of the polypeptide fragments according to the invention comprise parenteral, oral, transmucosal, topical, ocular, and transdermal. Pharmaceutical compositions as described above may thus be formulated for any appropriate manner of administration, including, for example, topical (e.g., transdermal or ocular), oral, buccal, nasal, vaginal, rectal or parenteral administration.

The term parenteral as used herein includes subcutaneous, intradermal, intravascular, or intravenous, intramuscular, spinal, intracranial, intrathecal, intraocular, periocular, intraorbital, intrasynovial and intraperitoneal injection, as well as any similar injection or infusion technique. The composition can be also for example a suspension, emulsion, sustained release formulation, cream, gel or powder. The composition can be formulated as a suppository, with traditional binders and carriers such as triglycerides.

In one example, the pharmaceutical formulation is a liquid formulation, *e*.*g*., a buffered, isotonic, aqueous solution. In another example, the pharmaceutical composition has a pH that is physiologic, or close to physiologic. In other examples, the aqueous formulation has a physiologic or close to physiologic osmolarity and salinity. It can contain sodium chloride and/or sodium acetate. In some examples, the composition of the present invention is lyophilized.

Still further are provided nucleic acid molecules encoding polypeptide fragments derived from fibrillin-1 having amino acid sequences as described above, expression vectors, recombinant host cells, transgenic animal cells, transgenic animals, process for the production of said polypeptide fragments, as well as fusion fragments obtainable by said process.

Nucleic acid molecules according to the present invention encode fibrillin-1 polypeptide fragments having the amino acid sequence as set forth in SEQ ID NO: 1, or at least 70%, 80%, 85%, 90%, or 95% identical thereto.

The terms "nucleic acid sequences" and "polynucleotides," used interchangeably herein, include polymeric forms of nucleotides of any length, including ribonucleotides, deoxyribonucleotides, or analogs or modified versions thereof. They include single-, double-, and multi-stranded DNA or RNA, genomic DNA, cDNA, DNA-RNA hybrids, and polymers comprising purine bases, pyrimidine bases, or other natural, chemically modified, biochemically modified, non-natural, or derivatized nucleotide bases. Nucleic acids are said to have "5' ends" and "3' ends" because mononucleotides are reacted to make oligonucleotides in a manner such that the 5' phosphate of one mononucleotide pentose ring is attached to the 3' oxygen of its neighbour in one direction via a phosphodiester linkage. An end of an oligonucleotide is referred to as the "5' end" if its 5' phosphate is not linked to the 3' oxygen of a mononucleotide pentose ring. An end of an oligonucleotide is referred to as the "3' end" if its 3' oxygen is not linked to a 5' phosphate of another mononucleotide pentose ring. A nucleic acid sequence, even if internal to a larger oligonucleotide, also may be said to have 5' and 3' ends. In either a linear or circular DNA molecule, discrete elements are referred to as being "upstream" or 5' of the "downstream" or 3' elements.

Said nucleic acid molecules may encode polypeptide fragments retaining the activity of fibrillin-1, which is (a) a nucleic acid molecule encoding a polypeptide comprising the amino acid sequence of SEQ ID NO: 1; (b) a nucleic acid molecule encoding fibrillin-1, the amino acid sequence of which is at least 60%, or at least 70% or at least 80% or at least 90%, at least 95% identical to the amino acid sequence of SEQ ID NO: 1; (c) a nucleic acid molecule comprising a nucleotide sequence which is at least 50% identical to the nucleotide sequence of SEQ ID NO: 1; (d) a nucleic acid molecule which is degenerate with respect to the nucleic acid molecule of (a to c); or (e) a nucleic acid molecule corresponding to the nucleic acid molecule of any one of (a) to (d) wherein at least one base is substituted with another. Further provided is a purified nucleic acid which: b) hybridizes under high stringency conditions; or c) exhibits at least about 85% identity over a stretch of at least about 10 to 30 nucleotides with a nucleic acid encoding polypeptide fragment of SEQ ID NO: 1.

It may be noted that the nucleic acids and the resulting polypeptides may comprise exogenous sequences and may be codon optimized for optimal expression in a cell system. "Exogenous" molecules or sequences include molecules or sequences that are not normally present in a cell in that form. Normal presence includes presence with respect to the particular developmental stage and environmental conditions of the cell. An exogenous molecule or sequence, for example, can include a mutated version of a corresponding endogenous sequence within the cell, such as a humanized version of the endogenous sequence, or can include a sequence corresponding to an endogenous sequence within the cell but in a different form (i.e., not within a chromosome). In contrast, endogenous molecules or sequences include molecules or sequences that are normally present in that form in a particular cell at a particular developmental stage under particular environmental conditions. "Codon optimization" generally includes a process of modifying a nucleic acid sequence for enhanced expression in particular host cells by replacing at least one codon of the native sequence with a codon that is more frequently or most frequently used in the genes of the host cell while maintaining the native amino acid sequence. For example, a polynucleotide can be modified to substitute codons having a higher frequency of usage in a given eukaryotic cell, including a bacterial cell, a yeast cell, a human cell, a non-human cell, a mammalian cell, a rodent cell, a mouse cell, a rat cell, a hamster cell, or any other host cell, as compared to the naturally occurring nucleic acid sequence. Codon usage tables are readily available, for example, at the "Codon Usage Database." See Nakamura et al. (2000) Nucleic Acids Research 28:292, herein incorporated by reference in its entirety for all purposes. Computer algorithms for codon optimization of a particular sequence for expression in a particular host are also available.

Expression vectors according to the present invention may comprise nucleotide sequences encoding the polypeptide fragments having the amino acid sequence as set forth in SEQ ID NO: 1, or at least 70%, at least 80%, at least 85%, at least 90%, at least 95% identical to the amino acid sequence as set forth in SEQ ID NO: 1, wherein the said nucleic acid molecule is operatively linked to expression control sequences allowing expression in eukaryotic host cells of the polypeptide.

As used herein "expression vector" refers to a discrete genetic element that is used to introduce heterologous nucleic acid into cells for either expression or replication thereof. Selection and use of such vehicles are well within the skill of the artisan. A recombinant expression vector includes vectors capable of expressing nucleic acids that are operatively linked to regulatory sequences, such as promoter regions that are capable of effecting expression of such nucleic acid sequences. Thus, an expression vector refers to a recombinant DNA or RNA construct, such as a plasmid, a phage, recombinant virus or other vector that, upon introduction into an appropriate host cell, results in expression of the cloned nucleic acid sequences. Appropriate expression vectors are well known to those of skill in the art and include those that are replicable in eukaryotic cells including those that remain episomal or those, which integrate into the host cell genome.

"Operable linkage" or being "operably linked" includes juxtaposition of two or more components (e.g., a promoter and another sequence element) such that both components function normally and allow the possibility that at least one of the components can mediate a function that is exerted upon at least one of the other components. For example, a promoter can be operably linked to a coding sequence if the promoter controls the level of transcription of the coding sequence in response to the presence or absence of one or more transcriptional regulatory factors. Operable linkage can include such sequences being contiguous with each other or acting in trans (e.g., a regulatory sequence can act at a distance to control transcription of the coding sequence).

A "promoter" is a regulatory region of DNA usually comprising a TATA box capable of directing RNA polymerase II to initiate RNA synthesis at the appropriate transcription initiation site for a particular polynucleotide sequence. A promoter may additionally comprise other regions which influence the transcription initiation rate.

The nucleic acid molecules may be inserted into several commercially available expression vectors. Non-limiting examples include prokaryotic plasmid vectors, such as the pUC-series, pBluescript (Stratagene), the pET-series of expression vectors (Novagen) or pCRTOPO (Invitrogen) and vectors compatible with an expression in mammalian cells like pREP (Invitrogen), pcDNA3 (Invitrogen), pCEP4 (Invitrogen), pMClneo (Stratagene), pXT1 (Stratagene), pSG5 (Stratagene), EBO-pSV2neo, pBPV-1, pdBPVMMTneo, pRSVgpt, pRSVneo, pSV2-dhfr, pIZD35, pLXIN, pSIR (Clontech), pIRES-EGFP (Clontech), pEAK-10 (Edge Biosystems) pTriEx-Hygro (Novagen) and pCINeo (Promega). Examples for plasmid vectors suitable for *Pichia pastoris* comprise e.g. the plasmids pAO815, pPIC9K and pPIC3.5K (all Intvitrogen). Non-limiting examples include pET32, pET41, pET43. The vectors may also contain an additional expressible polynucleotide coding for one or more chaperones to facilitate correct protein folding. Suitable bacterial expression hosts comprise e.g. strains derived from BL21 (such as BL21(DE3), BL21(DE3)PlysS, BL21(DE3)RIL, BL21(DE3)PRARE) or Rosetta^{®}. In preferred embodiments, expression vector according to the invention is a DNA or RNA vector, capable of transforming eukaryotic host cells and effecting stable or transient expression of said TRP channel fusion subunit, and wherein said vector is a plasmid, a virus like adenovirus, adeno associated virus (AVV), lentiviral, Epstein-Barr, Herpes Simplex, Papilloma, Polyoma, Retro, SV40, Vaccinia, any retroviral vector, influenza viral vector and other non-viral vectors including naked DNA, or liposomes. Other examples of viral vectors include, but are not limited to, nucleic acid sequences from the following viruses: retrovirus, such as Moloney murine leukemia virus, Harvey murine sarcoma virus, murine mammary tumor virus, and Rous sarcoma virus; adenovirus, adeno-associated virus; SV40-type viruses; polyomaviruses; Epstein-Barr viruses; papilloma viruses; herpes virus; vaccinia virus; polio virus; and RNA virus such as a retrovirus. One can readily employ other vectors well-known in the art. Certain viral vectors are based on non-cytopathic eukaryotic viruses in which non-essential genes have been replaced with the gene of interest. Non-cytopathic viruses include retroviruses, the life cycle of which involves reverse transcription of genomic viral RNA into DNA with subsequent proviral integration into host cellular DNA. Retroviruses have been approved for human gene therapy trials. Most useful are those retroviruses that are replication-deficient (i.e., capable of directing synthesis of the desired proteins, but incapable of manufacturing an infectious particle). Such genetically altered retroviral expression vectors have general utility for the high-efficiency transduction of genes *in vivo.* Standard protocols for producing replication-deficient retroviruses (including the steps of incorporation of exogenous genetic material into a plasmid, transfection of a packaging cell line with plasmid, production of recombinant retroviruses by the packaging cell line, collection of viral particles from tissue culture media, and infection of the target cells with viral particles) are provided in Kriegler, M., Gene Transfer and Expression, A Laboratory Manual, W.H. Freeman Co., New York (1990) and Murry, E. J., Methods in Molecular Biology, Vol. 7, Humana Press, Inc., Cliffton, N.J. (1991).

Transcriptional regulatory elements (parts of an expression cassette) ensuring expression in prokaryotes or eukaryotic cells are well known to those skilled in the art. These elements comprise regulatory sequences ensuring the initiation of the transcription (e.g., translation initiation codon, promoters, enhancers, and/or insulators), internal ribosomal entry sites (IRES) and optionally poly-A signals ensuring termination of transcription and stabilization of the transcript. Additional regulatory elements may include transcriptional as well as translational enhancers, and/or naturally associated or heterologous promoter regions. Expression vectors may further comprise nucleotide sequences encoding secretion signals as further regulatory elements. Such sequences are well known to the person skilled in the art. Furthermore, depending on the expression system used, leader sequences capable of directing the expressed polypeptide fragments to a cellular compartment may be added to the coding sequence of the polynucleotide of the invention. Such leader sequences are well known in the art.

A gene expression control sequence as used herein is any regulatory nucleotide sequence, such as a promoter sequence or promoter-enhancer combination, which facilitates the efficient transcription and translation of the coding nucleic acid to which it is operably linked. The gene expression control sequence may, for example, be a mammalian or viral promoter, such as a constitutive or inducible promoter. Constitutive mammalian promoters include, but are not limited to, the promoters for the following genes: hypoxanthine phosphoribosyl transferase (HPRT), adenosine deaminase, pyruvate kinase, beta-actin promoter, and other constitutive promoters. Exemplary viral promoters which function constitutively in eukaryotic cells include, for example, promoters from the cytomegalovirus (CMV), simian virus (e.g., SV40), papilloma virus, adenovirus, human immunodeficiency virus (HIV), Rous sarcoma virus, cytomegalovirus, the long terminal repeats (LTR) of Moloney leukemia virus, and other retroviruses, and the thymidine kinase promoter of herpes simplex virus. Other constitutive promoters are known to those of ordinary skills in the art. The promoters useful as gene expression sequences of the invention also include inducible promoters. Inducible promoters are expressed in the presence of an inducing agent. For example, the metallothionein promoter is induced to promote transcription and translation in the presence of certain metal ions. Other inducible promoters are known to those of ordinary skills in the art.

Possible examples for regulatory elements ensuring the initiation of transcription comprise the cytomegalovirus (CMV) promoter, SV40-promoter, RSV-promoter (Rous sarcome virus), the lacZ promoter, human elongation factor 1α-promoter, CMV enhancer, CaM-kinase promoter, the *Autographa californica* multiple nuclear polyhedrosis virus (AcMNPV) polyhedral promoter or the SV40-enhancer. For the expression in prokaryotes, a multitude of promoters including, for example, the tac-lac-promoter, the lacUV5 or the trp promoter, has been described. Examples for further regulatory elements in prokaryotes and eukaryotic cells comprise transcription termination signals, such as SV40-poly-A site or the tk-poly-A site or the SV40, lacZ and AcMNPV polyhedral polyadenylation signals, downstream of the polynucleotide.

The present invention is further directed to a recombinant host cell containing an expression vector for expression of polypeptide fragments as described above. Recombinant cells according to the present invention are thus genetically engineered with said expression vectors to express said polypeptides fragments. The cell or host may be any prokaryote or eukaryotic cell and may be preferably as stable cell line. Suitable eukaryotic host may be a mammalian cell, an amphibian cell, a fish cell, an insect cell, a fungal cell or a plant cell. Eukaryotic cell may be an insect cell such as a *Spodoptera frugiperda* cell, a yeast cell such as a *Saccharomyces cerevisiae* or *Pichia pastoris* cell, a fungal cell such as an *Aspergillus* cell or a vertebrate cell. Suitable prokaryotes may be *E. coli* (e.g., *E coli* strains HB101, DH5a, XL1 Blue, Y1090 and JM101), *Salmonella typhimurium, Serratia marcescens, Burkholderia glumae, Pseudomonas putida, Pseudomonas fluorescens, Pseudomonas stutzeri, Streptomyces lividans, Lactococcus lactis, Mycobacterium smegmatis* or *Bacillus subtilis.*

The expression vector or vectors are then transfected or co-transfected into a suitable target cell, which will express the polypeptides. Transfection techniques known in the art include, but are not limited to, calcium phosphate precipitation, electroporation, and liposome-based reagents. A variety of host-expression vector systems may be utilized to express the proteins described herein including eukaryotic cells. These include, but are not limited to, microorganisms such as bacteria (e.g., E. coli) transformed with recombinant bacteriophage DNA or plasmid DNA expression vectors containing an appropriate coding sequence; yeast or filamentous fungi transformed with recombinant yeast or fungi expression vectors containing an appropriate coding sequence; insect cell systems infected with recombinant virus expression vectors (e.g., baculovirus) containing an appropriate coding sequence; plant cell systems infected with recombinant virus expression vectors (e.g., cauliflower mosaic virus or tobacco mosaic virus) or transformed with recombinant plasmid expression vectors (e.g., Ti plasmid) containing an appropriate coding sequence; or animal cell systems, including mammalian cells (e.g., HEK 293, CHO, Cos, HeLa, HKB11, and BHK cells).

Typically host cells are eukaryotic cells and may include for example cells of vertebrates, e.g., mammals, and cells of invertebrates, e.g., insects. Eukaryotic cells of plants specifically can include, without limitation, yeast cells. Eukaryotic cells may be mammalian cells, such as human cells, such as for example mammalian cell line HEK 293 cell, which is a human embryonic kidney cell line. HEK 293 cells are available as CRL-1533 from American Type Culture Collection, Manassas, Va., and as 293-H cells, Catalog No. 11631-017 or 293-F cells, Catalog No. 11625-019 from Invitrogen (Carlsbad, Calif.). Alternatively, mammalian cells may be PER.C6^{®} cell, which is a human cell line derived from retina. PER.C6^{®} cells are available from Crucell (Leiden, The Netherlands), or Chinese hamster ovary (CHO) cell (CHO cells are available from American Type Culture Collection, Manassas, Va. Under Accession No. CHO-K1; CCL-61), or baby hamster kidney (BHK) cell (BHK cells are available from American Type Culture Collection, Manassas, Va. under accession No. CRL-1632), or HKB11 cell, which is a hybrid cell line of a HEK293 cell and a human B cell line. Host cells are stably transfected, so that they can be selected and maintained as a stable cell line, using conventional techniques known to those of skill in the art.

Recombinant host cells which contain nucleic acid molecules encoding polypeptide fragments are grown in an appropriate growth medium. As used herein, the term "appropriate growth medium" means a medium containing nutrients required for the growth of cells. Nutrients required for cell growth may include a carbon source, a nitrogen source, essential amino acids, vitamins, minerals, and growth factors. Optionally, the media can contain one or more selection factors. Cultured mammalian cells are generally grown in commercially available serum-containing or serum-free media (e.g., MEM, DMEM, DMEM/F12). Examples of appropriate medium include CD293 (Invitrogen, Carlsbad, Calif.), or CD17 (Invitrogen, Carlsbad, Calif.). Selection of a medium appropriate for the particular cell line used is within the level of those ordinary skilled in the art.

The process for producing polypeptide fragments according to the present invention may thus comprise genetically engineering recombinant cells with a vector or a nucleic acid encoding said polypeptide fragments under conditions in which the nucleic acid or vector is expressed, and recovering the polypeptide fragments encoded by said nucleic acid or vector from the culture. Transgenic animal cells may be transformed with a vector or a nucleic acid molecule according to the present invention, having enhanced or reduced expression levels of the polypeptide fragments of the invention.

*In vitro* production allows scale-up to give large amounts of the desired altered polypeptide fragments of the invention. Techniques for mammalian cell cultivation under tissue culture conditions are known in the art and include homogeneous suspension culture, e.g., in an airlift reactor or in a continuous stirrer reactor, or immobilized or entrapped cell culture, e.g., in hollow fibers, microcapsules, on agarose microbeads or ceramic cartridges. If necessary and/or desired, the solutions of polypeptides can be purified by the customary chromatography methods, for example gel filtration, ion-exchange chromatography, hydrophobic interaction chromatography (HIC, chromatography over DEAE-cellulose or affinity chromatography

The practice of the present invention will employ, unless otherwise indicated, conventional techniques of cell biology, cell culture, molecular biology, transgenic biology, microbiology, recombinant DNA, and immunology, which are within the skill of the art. Such techniques are explained fully in the literature. See, for example, Molecular Cloning A Laboratory Manual, 2nd Ed., Sambrook et al., ed., Cold Spring Harbor Laboratory Press: (1989); Molecular Cloning: A Laboratory Manual, Sambrook et al., ed., Cold Springs Harbor Laboratory, New York (1992), DNA Cloning, D. N. Glover ed., Volumes I and II (1985); Oligonucleotide Synthesis, M. J. Gait ed., (1984); Mullis et al. U.S. Pat. No. 4,683,195; Nucleic Acid Hybridization, B. D. Hames & S. J. Higgins eds. (1984); Transcription And Translation, B. D. Hames & S. J. Higgins eds. (1984); Culture Of Animal Cells, R. I. Freshney, Alan R. Liss, Inc., (1987); Immobilized Cells And Enzymes, IRL Press, (1986); B. Perbal, A Practical Guide To Molecular Cloning (1984); the treatise, Methods In Enzymology, Academic Press, Inc., N.Y.; Gene Transfer Vectors For Mammalian Cells, J. H. Miller and M. P. Calos eds., Cold Spring Harbor Laboratory (1987); Methods In Enzymology, Vols. 154 and 155 (Wu et al. eds.); Immunochemical Methods In Cell And Molecular Biology, Mayer and Walker, eds., Academic Press, London (1987); Handbook Of Experimental Immunology, Volumes I-IV, D. M. Weir and C. C. Blackwell, eds., (1986); Manipulating the Mouse Embryo, Cold Spring Harbor Laboratory Press, Cold Spring Harbor, N.Y., (1986); and in Ausubel et al., Current Protocols in Molecular Biology, John Wiley and Sons, Baltimore, Md. (1989). Standard reference works setting forth general principles of immunology include Current Protocols in Immunology, John Wiley & Sons, New York; Klein, J., Immunology: The Science of Self-Nonself Discrimination, John Wiley & Sons, New York (1982); Roitt, I., Brostoff, J. and Male D., Immunology, 6thed. London: Mosby (2001); Abbas A., Abul, A. and Lichtman, A., Cellular and Molecular Immunology, Ed. 5, Elsevier Health Sciences Division (2005); and Harlow and Lane, Antibodies: A Laboratory Manual, Cold Spring Harbor Press (1988).

Throughout this application, various references are referred to and disclosures of these publications in their entireties are hereby incorporated by reference into this application to more fully describe the state of the art to which this invention pertains.

### EXAMPLES

### Example 1: Detection of Fibrillin-1 in the developing retinal vasculature but with a restricted spatial localization

Fibrillin-1 was detected in the developing retinal vasculature by immunofluorescent staining of flat mounted wild type mouse retinas at P6 (Figure 1A). In endothelial marker isolectin B4 (IB4)-stained retinas, the protein was found associated with arterioles growing from the optic nerve head but unexpectedly, also at endothelial sprouts headed by tip cells (Figure 1B). Fibrillin-1-specific localization at the angiogenic front was verified in P2, P4 and P6 retinas (data not shown). At higher magnification, fibrillin-1 was observed around arterioles but detected rather as matrix deposits in the tip cell surroundings and along tip cell bodies and their projections (Figure 1C). Elastin staining confirmed an arteriole-associated distribution of fibrillin-1 (Figure ID, bottom panels). However, elastin was not detected at the sprouting area of the angiogenic front where fibrillin-1 was abundant (Figure ID top panels). In fibrillin-1 mutant mice tested at P6, fibrillin-1 immunostaining at the angiogenic front (Figure 2A) and at arterioles (Figure 2B), was reduced compared to wild type mice, reflecting the reported fibrillin-1 haploinsufficiency in this model. The retinal vasculature of MFS mice was explored in more detail at fibrillin-1 enriched topographical locations

### Example 2: Determination that mutations in fibrillin-1 reduced developmental angiogenesis in the retina

During the initial expansion phase of retinal vasculature development, the overall diameter of the plexus covering the retinal surface was an indicator of decreased or increased angiogenesis. Analysis of flat-mounted retinas at P6 stained for IB4 revealed that both the vascular coverage of the retina and the radial extension of the network were significantly reduced in the Fbn1^{C1041G/+} mice, as compared to wild type littermate controls (Figure 3A). At the angiogenic front, the density of capillaries, the number of sprouts, capillary branch points and meshes were also decreased in Fbn1^{C1041G/+} mice (Figure 3B). Tip cells use their filopodia to adhere to the matrix for cell migration and podosomes facilitate this process by degrading the BM during the sprouting, branching and anastomosing steps of angiogenesis. The reduction in vascular outgrowth toward the retinal periphery suggested a migration defect. As the number of sprouts/tip cells was reduced in mutant retinas, the line demarcating the angiogenic front appeared less indented (Figure 3B, C) but the number of filopodia per vascular segment length was globally reduced (Figure 3C). Impaired sprouting and branching of Fbn1^{C1041G/+} retinal vessels could also result from a defect in podosome formation. The structures form clusters intermingled with the subcortical actin filament meshwork at the distal tip cell end where filipodia sprout from the leading edge. Lifeact-EGFP-transgenic mice, in which the expression of the transgene is largely restricted to the endothelium was used to facilitate their detection by cortactin and Col IV labelling as described by Spuul et al. (Cell Rep 2016 Oct 4;17(2):484-500; doi: 10.1016/j.celrep.2016.09.016). Maximum intensity projections of confocal Z-stacks showed that podosomes coincided with gaps in Col IV staining (Figure 4A, B). A significant reduction was observed in podosome density and podosome size in P6 Lifeact-EGFP/Fbn1^{C1041G/+} retinas (Figure 4A). Quantification of BM-Col IV staining at the level of tip cells revealed increased protein coverage as compared to control mice (Figure 4A, B). Collectively, these results indicated reduced podosome formation and suggested decreased proteolytic activity at the angiogenic front in the developing retinal vasculature of Fbn1^{C1041G/+} animals.

### Example 3: Determining that the Fbn1^{C1041G/+} mutation affected the proliferation of endothelial cells

As reduced vascular density is often associated with decreased stalk cell proliferation, overall vascular density was addressed by quantifying the EC transcription factor ERG1/2/3 and the mitotic marker phospho-histone-H3 (PH3). Both signals were significantly reduced in the vascular plexus of mutant mice (Figure 5A, B). Thus, mutant fibrillin-1 impaired vascularization at both the angiogenic front and primary plexus.

### Example 4: Determining that the Fbn1^{C1041G/+} mutation affected the retinal arteriole integrity

The mature retinal vasculature was then analysed. Artery/vein differentiation was particularly noticeable in the retinal vasculature because of the radially alternating patterning of arterioles and venules (Figure 1A). A progressive enlargement of the retinal arterioles in Fbn1^{C1041G/+} mice was observed as compared to wild type littermate controls (Figure 6A-D). In contrast, the vein diameter was found unaffected (Figure 7C). No arteriovenous malformation which is often associated with arteriole enlargement have been detected. To investigate the causes of arteriole enlargement, it was first examined whether the C1041G mutation in fibrillin-1 affected contractile VSMC coverage. Data in Results of Figure 6A-C showed that retinal arterioles displayed uniform immunoreactivity for the contractile VSMC marker αSMA, and for the endothelial cell marker IB4 in wild type retinas (left panels). In P6, 4 months and 1 year old Fbn1^{C1041G/+} mice, a focal loss of αSMA coverage along retinal arterioles was apparent, whereas IB4 staining remained uniform (right panels). Quantitative analysis of these parameters showed that the heterozygous C1041G mutation resulted in a loss of contractile VSMC coverage and an increase in the number of αSMA-deficient gaps as compared to retinal arterioles (Figure 6E). To explore if these lesions were associated with functional defects, an immunostaining for albumin was performed. Albumin was not detected around vessels in control wild type retinas (Figure 6 B, C, left panels) but representative images from 4 months and 1 year old Fbn1^{C1041G/+} mice (Figure 6B, C, right panels) showed diffuse albumin staining around arterioles and more particularly in regions corresponding to poor VSMC coverage, indicating plasma protein extravasation from leaky vessels in Fbn1^{C1041G/+} retinas (Figure 6F).

Moreover, quantitative analysis of Col IV and laminin (γ1 chain) staining showed reduced overall levels in retinal arterioles from 4 months old Fbn1^{C1041G/+} mice in comparison with wild type mice (Figure 7A, B), indicating altered basement membrane integrity. Collectively, these results suggested that altered BM integrity and focal or segmental loss of VSMCs were involved in vessel permeability detected in the retinas from Fbn1^{C1041G/+} mice.

### Example 5: In vitro pro-angiogenic properties of a human fibrillin-1 fragment hfrag¹⁴⁸⁷⁻²⁷²⁵

Based on the evidence of the generated data that suggested that fibrillin-1 contributes to sprouting angiogenesis and vascular branching, recombinants human C-term or N-term fibrillin-1 sub-fragments (hfrag¹⁴⁸⁷⁻²⁷²⁵ and hfrag¹⁷⁻¹⁵²⁷) were tested in *in vitro* assays that mimicked the context of tip-cell biogenesis. A Matrigel plug containing VEGF-A was placed in a culture dish and primary endothelial cells (human microvascular endothelial cells, HMVECs) were seeded to form a monolayer around it (Figure 8A). At the cell monolayer-Matrigel interface, tip cell-like endothelial cells assembled podosomes, projected filopodia toward the VEGF-A source and invaded the Matrigel. Endothelial cell sprouting was selectively stimulated when the Matrigel was supplemented with the human fibrillin-1 fragment hfrag¹⁴⁸⁷⁻²⁷²⁵ (Figure 8B). Likewise, podosome formation was more robust as reflected by the increase in podosome number and size in this situation, as compared to controls (Figure 8C). Human fibrillin-1 fragment hfrag¹⁴⁸⁷⁻²⁷²⁵ was then tested in an *in vitro* assay mimicking the formation of a primitive microvascular network. HMVECs were seeded on Matrigel-coated cell culture dishes and allowed to spontaneously form interconnected capillary-like structures (Figure 9A). This process was selectively enhanced when the Matrigel was supplemented with hfrag¹⁴⁸⁷⁻²⁷²⁵ (Figure 9B). Taken together, these results established that endothelial cells were targets for fibrillin-1 fragment of the present invention and that these fragments displayed pro-angiogenic effects on endothelial cells by stimulating sprouting and branching.

### Example 6: Murine fibrillin-1 fragment mfrag¹⁴⁸⁹⁻²⁷²⁷ restored the retinal vascular defects of Fbn1^{C1041G/+} mice

In an attempt to overcome the fibrillin-1 deficiency caused by the C1041G mutation. Intra-vitreal injection of mfrag¹⁴⁸⁹⁻²⁷²⁷ was performed in the eye of WT and Fbn1^{C1041G/+} animals at P4 (Figure 10A). Neo-vascularisation was allowed until P6 and angiogenic parameters were assessed. Data presented in Figures 10, 11 show that angiogenesis was restored under these conditions. Vascular coverage of the retina, radial extension and vascular density of the network were stimulated to reach wild type littermate control values in mfrag¹⁴⁸⁹⁻²⁷²⁷-injected eyes from Fbn1^{C1141G1+} mice (Figure 10B). Importantly, fibrillin binding MAGP1 immunostaining, which appeared weaker in Fbn1^{C1041G/+} vs control retinas, was restored and reached control values in mfrag¹⁴⁸⁹⁻²⁷²⁷ injected Fbn1^{C1041G/+} retinas (Figure 11A). Moreover, Col IV coverage, which appeared increased in Fbn1^{C1041G/+} vs control retinas, was partially restored in mfrag¹⁴⁸⁹⁻²⁷²⁷ injected Fbn1^{C1041G/+} eyes (Figure 11A). It was also addressed whether the mfrag¹⁴⁸⁹⁻²⁷²⁷ fragment administration had an effect on more mature vessels such as the retinal arteriole. Data presented in Figure 11C showed that arteriolar diameter and MAGP1 expression were normalized in mfrag¹⁴⁸⁹⁻²⁷²⁷-injected eyes from Fbn1^{C1041G/+} mice.

Taken together, the data show that the murine fibrillin-1 fragment mfrag¹⁴⁸⁹⁻²⁷²⁷ according to the present invention rescued most defects resulting from defective fibrillin-1 during vascularisation of the retina in the Fbn1^{C1041G/+} model of Marfan mice. They also revealed the proangiogenic effect on endothelial cells and the protective effect on mature vessels of the fragment.

### Example 7: Human fibrillin-1 fragment hfrag¹⁴⁸⁷⁻²⁷²⁵ restored the retinal vascular defects of Fbn1^{C1041G/+} mice

Intra-vitreal injection of the hfrag¹⁴⁸⁷⁻²⁷²⁵ fragment was performed in the eye of WT and Fbn1^{C1041G/+} animals at P4 (Figure 12A). Neo-vascularisation was then allowed until P6 and angiogenic parameters were assessed. Data presented in Figures 12, 13 showed that the human fibrillin-1 fragment hfrag¹⁴¹⁷⁻²⁷²⁵ exerted similar effects than the mfrag¹⁴⁸⁹⁻²⁷²⁷ fragment. As for the murine fragment, both angiogenesis (Figure 12B, 13A) and arteriole integrity (Figure 13B) were restored in hfrag¹⁴⁸⁷⁻²⁷²⁵ injected retinas from Fbn1^{C1041G/+} mice. These data suggest that both fragments exerted proangiogenic and vascular protective effects.

### Experimental Procedures

### Mice

Lifeact-EGFP transgenic mice and Fbn1^{C1041G/+} mice were used in the above Examples. Both strains were maintained on a C57BL/6 background. Fbn1^{C1041G/+} mice correspond to the B6.129-Fbn1tm1Hcd/J mouse strain (012885 - B6.129-Fbn1<tm1Hcd>/J (jax.org)), sometimes referenced in the literature as Fbn1^{C1039G/+} mice. The two names coexist in the literature depending if we refer to the murine or the human mutation, respectively.

All experimental procedures with mice were performed using protocols approved by the Committee for Ethics of Animal Experiments at the University of Bordeaux, in accordance with the guidelines of the French Ministry of Health under the authority of the project License #7313. For administration of the murine and human FBN1 C-terminal recombinant fragments WT and Fbn1^{C1041G/+} pups were intravitreally injected into the left eye with 1 µL mfrag¹⁴⁸⁹⁻²⁷²⁷ or hfrag¹⁴⁸⁷⁻²⁷²⁵ reconstituted in sterile TBS to a final concentration of 0.8 µg/µl. Injections were performed using a 33-gauge needle placed on a 10-µl Nanofil syringe controlled by a UMP3 pump controller (World Precision Instruments) as previously described. TBS was injected into the contralateral eye as control. 48 h after injections, the animals were euthanized, the eyes were removed, and retinal flat mounts were analysed. Male and female mice were not distinguished until P6, and male mice were used at 4 month, and one-year. The numbers of analysed mice were indicated in each experiment.

### Production of fibrillin-1 fragments

Murine fibrillin-1 fragments (mfrag¹⁴⁸⁹⁻²⁷²⁷), human fibrillin-1 fragments (hfrag¹⁴⁸⁷⁻²⁷²⁵) and the N-terminal half of human fibrillin1 (hfrag¹⁷⁻¹⁵²⁷) were recombinantly expressed. Briefly, the recombinant proteins were expressed in human embryonic kidney cells, and subsequently purified by immobilized metal ion affinity chromatography. N-terminal sequencing validated the correct sequence of the recombinant proteins.

### Whole retina immunohistochemistry

P6, 4-months and 1-year old mice were sacrificed, enucleated and the eyes were fixed in 4% paraformaldehyde for 2 h at 4°C. Retinas were dissected, then incubated for 2 h at room temperature in blocking buffer (PBS, 2% BSA, 0.2% Triton X-100). After three 20 min washes in Pblec buffer (PBS supplemented with 1 mM MgCl2, 1 mM MnCl2, 1 mM CaCl2 and 1% Triton X- 100), retinas were incubated overnight at 4°C with fluorescein labelled isolectin B4 (IB4; Vector Laboratories, FL-1201, 1:25) and antibodies diluted in blocking buffer. Retinas were washed 3 times with blocking buffer and incubated with species-specific Alexa488-, 546- or 647-labeled secondary antibodies (Jackson Laboratories, 1:100) diluted in blocking buffer for 2 h at room temperature. After 3 washes in PBS, whole retinas were flat-mounted in ProLong Gold Antifade reagent (Life Technologies) containing Hoechst 33342, and analysed with an epifluorescence microscope (Nikon TE-2000) or a laser scanning fluorescence microscope (Zeiss LSM 510 Meta inverted).

### Antibodies for immunohistochemistry

A polyclonal antiserum against mouse fibrillin-1 was produced in rabbit. Anti-fibrillin-1 antibodies were affinity-purified and antibodies cross-reacting with fibronectin were removed by absorption resulting in monospecific anti-fibrillin-1 antibodies. Antibodies against Cortactin (clone 4F11, #05-180) and PH3 (#06-570) were obtained from Millipore. Antibodies against ERG 1/2/3 (ab92513) were from Abcam. Laminin gamma-1 (3E10) Antibodies (sc-65643) were purchased at Santa Cruz Biotechnology, Inc. Antibodies against SMA (clone 1A4, C6198) were purchased from Sigma. Col IV antibodies were obtained from BioRad (#2150-1470). Antibodies against MAGP1 (AF4977) and Serum Albumin (AF3329) were purchased from R&D Systems. Tropoelastin antibodies were a gift from Dr. Robert P. Mecham. For secondary detection, species-specific Alexa Fluor 488-, 546- or 647-labeled secondary antibodies (Jackson Laboratories) were used.

### Analysis of the neonatal retinal vasculature

To quantify vascular progression within neonatal retinas, mosaic images of IB4-labeled retinas were obtained by stitching individual images acquired at 4× magnification with a fluorescence microscope (Nikon TE-2000) using the 'large image' function of the NIS-elements software. From these pictures, the vascular coverage (IB4-positive retina area normalized to the total retina area), the radial extension of the vessels (distance from the optic nerve to the extremity of the network; measured in each quarter of the retinas), and the vascular density at the angiogenic front (percentage of IB4-positive areas per field, determined in ROIs from each quarter of the retinas) were quantified using the ImageJ software. The number of sprouts, tip cells and filopodia per ROI at angiogenic fronts was determined by manual counting. The geometry of the vascular network at angiogenic fronts (number of branch points and meshes) was automatically analyzed using the Angiogenesis analyzer plug-in on ImageJ software.

To quantify endothelial cell proliferation at angiogenic fronts, z-stack confocal series of 5 optical sections (x/y/z = 575.85 × 575.85 × 2 µm) were acquired with a Zeiss LSM Meta 510 confocal microscope equipped with a 10× objective. The number of IB4+PH3+ and ERG1/2/3+ cells per field in sprouting areas at the vascular front was automatically determined using the ImageJ "Analyze Particles" function, performed on maximal intensity projections of the confocal stacks.

To quantify podosome number, average podosome size and Col-IV coverage at angiogenic fronts, zstack confocal series of 20 optical sections (x/y/z = 146.25 × 146.25 × 0.15 µm) were acquired with a 63 × oil immersion objective. Acquisitions were performed at the leading front of the developing retinal vasculature. To determine the density of podosomes per µm2 of endothelial cell, the mean number and size of podosomes (F-actin/cortactin-positive (yellow) foci) per vessel length was calculated using the ImageJ "Analyze Particles" function performed on maximal intensity projections of the confocal stacks after deconvolution (AutoQuant X3 software) and normalization to the Lifeact-EGFP-positive surface area (determined from the thresholded image of the corresponding channel). To determine Col IV coverage of retinal vessels, the Col IV-positive surface area per field was determined on thresholded images and normalized to the Lifeact-EGFP-positive surface area as previously described.

To quantify FBN1 and MAGP1 at the vascular front, z-stack confocal series of 20 optical sections (x/y/z = 182.81 × 182.81 × 0.25 µm) were acquired using a 63× oil immersion objective. The positive surface areas determined from the thresholded maximal intensity projections of the confocal stacks were quantified using the ImageJ software and normalized to the IB4- positive areas.

### Cells and cell culture

Human Pulmonary Microvascular Endothelial cells (HMVECs, Lonza) were maintained in complete endothelial cell growth medium (EGM-MV; Promocell, a culture medium that does not contain VEGF) containing antibiotics at 37°C in a 5% CO2 humidified atmosphere and used between passages 2 and 7. For stimulation, human recombinant VEGF-A (used at 25 ng/ml in all experiments, unless otherwise indicated) was obtained from Promocell.

### Angiogenesis invasion assay (AIA)

A cut yellow tip was placed centrally in a 3.5cm MatTek dish (MatTek Corporation, USA) and filled with 50 µl of pure growth-factor-reduced Matrigel^{™} (around 9 mg/ml) plus VEGF-A to create a BM like matrix barrier, as described by Spuul et al. (Cell Rep 2016 Oct 4;17(2):484-500; doi: 10.1016/j.celrep.2016.09.016). In some experiments, the Matrigel + VEGF plug was supplemented with the human C-terminal recombinant fibrillin-1 fragment hfrag¹⁴⁸⁷⁻²⁷²⁵ or with the human N-terminal recombinant fragment hfrag¹⁷⁻¹⁵²⁷ at a final concentration of 50 µg/mL. Matrigel plugs were then allowed to polymerize for 30 min at 37°C. After this step, the yellow pipette tip was carefully removed. Cells (5x10⁴) were seeded around to create a circular area around the Matrigel. Cells were allowed to adhere for 90 min at 37°C and 2 ml of complete medium were then layered on top. The dish was returned to 37°C for 12 hours. Then cells in AIA were fixed in 2% PFA for 30 min followed by quenching with 50 mM NH4Cl and permeabilization in 0.2% Triton X-100 for 1h and blocking in 3% BSA. Primary monoclonal antibodies against cortactin (clone 4F11, #05-180, Millipore) were used at a 1/100 dilution in blocking buffer. After 16 hours at 4°C cells were washed extensively in PBS, then an Alexa fluor-546 coupled secondary antibody against mouse IgG was added at 1/100 dilution in blocking buffer together with Alexa fluor-488 labelled phalloidin and DAPI for 3h at room temperature. The system was then mounted with anti-fade reagent (Molecular Probes).

To quantify the number of sprouts invading the Matrigel plug, mosaic images were obtained by stitching individual phase-contrast images acquired at 4× magnification with an automated microscope (Nikon TE-2000) using the 'large image' function of the NIS-elements software. To quantify podosome number and average podosome size in endothelial cells, z-stack confocal series of 10 optical sections (x/y/z = 146.25 × 146.25 × 0.15 µm) were acquired using a laser scanning fluorescence microscope (Zeiss LSM 510 Meta inverted) equipped with a 63× oil immersion objective. The mean number and size of podosomes (F-actin/cortactin-positive (yellow) foci) per sprout was calculated using the ImageJ "Analyze Particles" function performed on maximal intensity projections of the confocal stacks.

### Analysis of the adult retinal vasculature

To quantify the diameters of retinal arterioles and venules and Col IV, Laminins, SMA, Serum Albumin, and MAGP1, z-stack confocal series of 30 optical sections (x/y/z = 146.25 × 146.25 × 0.3 µm) with a 63× oil immersion objective. Acquisitions were performed at the area of the first collateral vessel bifurcation starting from the optic disc. The maximal intensity projections of the confocal stacks were analyzed using the ImageJ software. The diameters of retinal arterioles and venules were calculated from 3 different length measurements performed perpendicularly to the vessel axis at the first collateral vessel bifurcation. The positive surface areas per field were measured on thresholded images and normalized to the IB4-positive surface area.

### Statistics

Statistical analysis was performed with GraphPad Prism 6 (GraphPad Software, Inc., San Diego. CA, USA). Data are presented as mean values ± SD (bars) and individual values (dots) (scatter plot with bar). Significance was determined by using the Student's t-test or the one-way ANOVA, followed by Tukey's post-tests. P values <0.05 were considered statistically significant.

## Claims

1. An isolated polypeptide comprising amino acid sequence as set forth in SEQ ID NO: 1, or at least 70%, at least 80%, at least 85%, at least 90%, or at least 95% identical to the amino acid sequence as set forth in SEQ ID NO:1.

2. A nucleic acid molecule encoding the polypeptide as defined in claim.

3. A vector comprising the nucleic acid of claim 2.

4. The vector of claim 3, wherein said nucleic acid molecule is operatively linked to expression control sequences allowing expression in eukaryotic host cells of the polypeptide.

5. The polypeptide according to claim 1 for use as a medicament.

6. The polypeptide according to claim 1 for use as pro-angiogenic drug or vessel normalization agent or drug.

7. A pharmaceutical composition comprising an efficient amount of the polypeptide according to claim 1 and at least one pharmaceutically acceptable carrier and/or vehicle.

8. The pharmaceutical composition of claim 10, further comprising an anticoagulant, an anti-thrombotic agent, an anti-platelet agent, a fibrinolytic agent, a hypolipidemic agent, an antihypertensive agent, and/or an anti-ischemic agent.

9. The polypeptide of claim 1 for use in a method of treating and/or preventing ischemia or ischemic diseases or vascular diseases.

10. The polypeptide for use in a method of claim 9, wherein said ischemic diseases are peripheral ischemic diseases, ischemic heart diseases, retinopathies, age-related macular degeneration (AMD), hemangiomas, varicose veins, aneurysms and cerebrovascular diseases/malformations, inflammation- and diabetes vascular disease.

11. The polypeptide for use in a method of claim 10, wherein said peripheral ischemic diseases are peripheral arterial diseases, peripheral occlusive arterial disease, lower limb artery ischemic diseases, distal limb ischemic diseases, thromboembolic disorders, venous thrombosis, deep vein thrombosis, thrombophlebitis, arterial embolism, or wound healing in diabetic patients.

12. The polypeptide for use in a method of claim 10, wherein said ischemic heart diseases are cardiovascular diseases coronary arterial disease, coronary arterial thrombosis, myocardial infarction, angina pectoris, acute coronary syndrome, atrial fibrillation, ischemic sudden death, or transient ischemic attack.

13. The polypeptide for use in a method of claim 9, wherein said ischemic diseases are diabetic retinopathy, ocular ischemia, ischemic cerebrovascular diseases, cerebral arterial thrombosis, cerebral embolism, kidney embolism, pulmonary embolism, and ischemia and/or thrombosis resulting from (a) prosthetic valves or other implants, (b) indwelling catheters, (c) stents, (d) cardiopulmonary bypass, (e) hemodialysis, or (f) other procedures in which blood is exposed to an artificial surface that promotes thrombosis.

14. The polypeptide for use in a method of any one of claims 9-13, wherein said polypeptide is administered via parenteral, oral, transmucosal, topical, ocular, or transdermal route of administration.

15. The polypeptide for use in a method of any one of claims 9-14, comprising further administering an additional pro-angiogenic or angiogenic agent, an anticoagulant, an anti-thrombotic agent, an anti-platelet agent, a fibrinolytic agent, a hypolipidemic agent, an antihypertensive agent, and/or an anti-ischemic agent.


SEQUENCE LISTING

<110>  INSERM
       UNIVERSITE DE BORDEAUX
       ROYAL INSTITUTION FOR THE ADVANCEMENT OF LEARNING/MCGILL
       UNIVERSITY

<120>  NOVEL RECOMBINANT FRAGMENTS OF FIBRILLIN-1 AND METHODS OF USE
       THEREOF

<130>  2020-FIBRILLIN

<140>  Not Yet Known
<141>  2020-12-23

<160>  1

<170>  PatentIn version 3.5

<210>  1
<211>  1239
<212>  PRT
<213>  HUMAN

<400>  1

Asp Val Asn Glu Cys Leu Asp Pro Thr Thr Cys Ile Ser Gly Asn Cys
1               5                   10                  15

Val Asn Thr Pro Gly Ser Tyr Ile Cys Asp Cys Pro Pro Asp Phe Glu
            20                  25                  30

Leu Asn Pro Thr Arg Val Gly Cys Val Asp Thr Arg Ser Gly Asn Cys
        35                  40                  45

Tyr Leu Asp Ile Arg Pro Arg Gly Asp Asn Gly Asp Thr Ala Cys Ser
    50                  55                  60

Asn Glu Ile Gly Val Gly Val Ser Lys Ala Ser Cys Cys Cys Ser Leu
65                  70                  75                  80

Gly Lys Ala Trp Gly Thr Pro Cys Glu Met Cys Pro Ala Val Asn Thr
                85                  90                  95

Ser Glu Tyr Lys Ile Leu Cys Pro Gly Gly Glu Gly Phe Arg Pro Asn
            100                 105                 110

Pro Ile Thr Val Ile Leu Glu Asp Ile Asp Glu Cys Gln Glu Leu Pro
        115                 120                 125

Gly Leu Cys Gln Gly Gly Lys Cys Ile Asn Thr Phe Gly Ser Phe Gln
    130                 135                 140

Cys Arg Cys Pro Thr Gly Tyr Tyr Leu Asn Glu Asp Thr Arg Val Cys

145                 150                 155                 160

Asp Asp Val Asn Glu Cys Glu Thr Pro Gly Ile Cys Gly Pro Gly Thr
                165                 170                 175

Cys Tyr Asn Thr Val Gly Asn Tyr Thr Cys Ile Cys Pro Pro Asp Tyr
            180                 185                 190

Met Gln Val Asn Gly Gly Asn Asn Cys Met Asp Met Arg Arg Ser Leu
        195                 200                 205

Cys Tyr Arg Asn Tyr Tyr Ala Asp Asn Gln Thr Cys Asp Gly Glu Leu
    210                 215                 220

Leu Phe Asn Met Thr Lys Lys Met Cys Cys Cys Ser Tyr Asn Ile Gly
225                 230                 235                 240

Arg Ala Trp Asn Lys Pro Cys Glu Gln Cys Pro Ile Pro Ser Thr Asp
                245                 250                 255

Glu Phe Ala Thr Leu Cys Gly Ser Gln Arg Pro Gly Phe Val Ile Asp
            260                 265                 270

Ile Tyr Thr Gly Leu Pro Val Asp Ile Asp Glu Cys Arg Glu Ile Pro
        275                 280                 285

Gly Val Cys Glu Asn Gly Val Cys Ile Asn Met Val Gly Ser Phe Arg
    290                 295                 300

Cys Glu Cys Pro Val Gly Phe Phe Tyr Asn Asp Lys Leu Leu Val Cys
305                 310                 315                 320

Glu Asp Ile Asp Glu Cys Gln Asn Gly Pro Val Cys Gln Arg Asn Ala
                325                 330                 335

Glu Cys Ile Asn Thr Ala Gly Ser Tyr Arg Cys Asp Cys Lys Pro Gly
            340                 345                 350

Tyr Arg Phe Thr Ser Thr Gly Gln Cys Asn Asp Arg Asn Glu Cys Gln
        355                 360                 365

Glu Ile Pro Asn Ile Cys Ser His Gly Gln Cys Ile Asp Thr Val Gly
    370                 375                 380

Ser Phe Tyr Cys Leu Cys His Thr Gly Phe Lys Thr Asn Asp Asp Gln
385                 390                 395                 400

Thr Met Cys Leu Asp Ile Asn Glu Cys Glu Arg Asp Ala Cys Gly Asn
                405                 410                 415

Gly Thr Cys Arg Asn Thr Ile Gly Ser Phe Asn Cys Arg Cys Asn His
            420                 425                 430

Gly Phe Ile Leu Ser His Asn Asn Asp Cys Ile Asp Val Asp Glu Cys
        435                 440                 445

Ala Ser Gly Asn Gly Asn Leu Cys Arg Asn Gly Gln Cys Ile Asn Thr
    450                 455                 460

Val Gly Ser Phe Gln Cys Gln Cys Asn Glu Gly Tyr Glu Val Ala Pro
465                 470                 475                 480

Asp Gly Arg Thr Cys Val Asp Ile Asn Glu Cys Leu Leu Glu Pro Arg
                485                 490                 495

Lys Cys Ala Pro Gly Thr Cys Gln Asn Leu Asp Gly Ser Tyr Arg Cys
            500                 505                 510

Ile Cys Pro Pro Gly Tyr Ser Leu Gln Asn Glu Lys Cys Glu Asp Ile
        515                 520                 525

Asp Glu Cys Val Glu Glu Pro Glu Ile Cys Ala Leu Gly Thr Cys Ser
    530                 535                 540

Asn Thr Glu Gly Ser Phe Lys Cys Leu Cys Pro Glu Gly Phe Ser Leu
545                 550                 555                 560

Ser Ser Ser Gly Arg Arg Cys Gln Asp Leu Arg Met Ser Tyr Cys Tyr
                565                 570                 575

Ala Lys Phe Glu Gly Gly Lys Cys Ser Ser Pro Lys Ser Arg Asn His
            580                 585                 590

Ser Lys Gln Glu Cys Cys Cys Ala Leu Lys Gly Glu Gly Trp Gly Asp
        595                 600                 605

Pro Cys Glu Leu Cys Pro Thr Glu Pro Asp Glu Ala Phe Arg Gln Ile
    610                 615                 620

Cys Pro Tyr Gly Ser Gly Ile Ile Val Gly Pro Asp Asp Ser Ala Val
625                 630                 635                 640

Asp Met Asp Glu Cys Lys Glu Pro Asp Val Cys Lys His Gly Gln Cys
                645                 650                 655

Ile Asn Thr Asp Gly Ser Tyr Arg Cys Glu Cys Pro Phe Gly Tyr Thr
            660                 665                 670

Leu Ala Gly Asn Glu Cys Val Asp Thr Asp Glu Cys Ser Val Gly Asn
        675                 680                 685

Pro Cys Gly Asn Gly Thr Cys Lys Asn Val Ile Gly Gly Phe Glu Cys
    690                 695                 700

Thr Cys Glu Glu Gly Phe Glu Pro Gly Pro Met Met Thr Cys Glu Asp
705                 710                 715                 720

Ile Asn Glu Cys Ala Gln Asn Pro Leu Leu Cys Ala Phe Arg Cys Val
                725                 730                 735

Asn Thr Tyr Gly Ser Tyr Glu Cys Lys Cys Pro Val Gly Tyr Val Leu
            740                 745                 750

Arg Glu Asp Arg Arg Met Cys Lys Asp Glu Asp Glu Cys Glu Glu Gly
        755                 760                 765

Lys His Asp Cys Thr Glu Lys Gln Met Glu Cys Lys Asn Leu Ile Gly
    770                 775                 780

Thr Tyr Met Cys Ile Cys Gly Pro Gly Tyr Gln Arg Arg Pro Asp Gly
785                 790                 795                 800

Glu Gly Cys Val Asp Glu Asn Glu Cys Gln Thr Lys Pro Gly Ile Cys
                805                 810                 815

Glu Asn Gly Arg Cys Leu Asn Thr Arg Gly Ser Tyr Thr Cys Glu Cys
            820                 825                 830

Asn Asp Gly Phe Thr Ala Ser Pro Asn Gln Asp Glu Cys Leu Asp Asn
        835                 840                 845

Arg Glu Gly Tyr Cys Phe Thr Glu Val Leu Gln Asn Met Cys Gln Ile
    850                 855                 860

Gly Ser Ser Asn Arg Asn Pro Val Thr Lys Ser Glu Cys Cys Cys Asp
865                 870                 875                 880

Gly Gly Arg Gly Trp Gly Pro His Cys Glu Ile Cys Pro Phe Gln Gly
                885                 890                 895

Thr Val Ala Phe Lys Lys Leu Cys Pro His Gly Arg Gly Phe Met Thr
            900                 905                 910

Asn Gly Ala Asp Ile Asp Glu Cys Lys Val Ile His Asp Val Cys Arg
        915                 920                 925

Asn Gly Glu Cys Val Asn Asp Arg Gly Ser Tyr His Cys Ile Cys Lys
    930                 935                 940

Thr Gly Tyr Thr Pro Asp Ile Thr Gly Thr Ser Cys Val Asp Leu Asn
945                 950                 955                 960

Glu Cys Asn Gln Ala Pro Lys Pro Cys Asn Phe Ile Cys Lys Asn Thr
                965                 970                 975

Glu Gly Ser Tyr Gln Cys Ser Cys Pro Lys Gly Tyr Ile Leu Gln Glu
            980                 985                 990

Asp Gly Arg Ser Cys Lys Asp Leu Asp Glu Cys Ala Thr Lys Gln His
        995                 1000                1005

Asn Cys Gln Phe Leu Cys Val Asn Thr Ile Gly Gly Phe Thr Cys
    1010                1015                1020

Lys Cys Pro Pro Gly Phe Thr Gln His His Thr Ser Cys Ile Asp
    1025                1030                1035

Asn Asn Glu Cys Thr Ser Asp Ile Asn Leu Cys Gly Ser Lys Gly
    1040                1045                1050

Ile Cys Gln Asn Thr Pro Gly Ser Phe Thr Cys Glu Cys Gln Arg
    1055                1060                1065

Gly Phe Ser Leu Asp Gln Thr Gly Ser Ser Cys Glu Asp Val Asp
    1070                1075                1080

Glu Cys Glu Gly Asn His Arg Cys Gln His Gly Cys Gln Asn Ile
    1085                1090                1095

Ile Gly Gly Tyr Arg Cys Ser Cys Pro Gln Gly Tyr Leu Gln His
    1100                1105                1110

Tyr Gln Trp Asn Gln Cys Val Asp Glu Asn Glu Cys Leu Ser Ala
    1115                1120                1125

His Ile Cys Gly Gly Ala Ser Cys His Asn Thr Leu Gly Ser Tyr
    1130                1135                1140

Lys Cys Met Cys Pro Ala Gly Phe Gln Tyr Glu Gln Phe Ser Gly

1145                1150                1155

Gly Cys Gln Asp Ile Asn Glu Cys Gly Ser Ala Gln Ala Pro Cys
    1160                1165                1170

Ser Tyr Gly Cys Ser Asn Thr Glu Gly Gly Tyr Leu Cys Gly Cys
    1175                1180                1185

Pro Pro Gly Tyr Phe Arg Ile Gly Gln Gly His Cys Val Ser Gly
    1190                1195                1200

Met Gly Met Gly Arg Gly Asn Pro Glu Pro Pro Val Ser Gly Glu
    1205                1210                1215

Met Asp Asp Asn Ser Leu Ser Pro Glu Ala Cys Tyr Glu Cys Lys
    1220                1225                1230

Ile Asn Gly Tyr Pro Lys
    1235